(19) Europäisches Patentamt
European Patent Office
Office européen des brevets

(11) Veröffentlichungsnummer : **0 506 867 B1**

# (12) EUROPÄISCHE PATENTSCHRIFT

(45) Veröffentlichungstag der Patentschrift : **20.09.95 Patentblatt 95/38**

(51) Int. Cl.[6] : **G03G 9/097,** G03G 9/10, G03G 9/12, C09D 7/12

(21) Anmeldenummer : **91902682.3**

(22) Anmeldetag : **17.12.90**

(86) Internationale Anmeldenummer : **PCT/EP90/02207**

(87) Internationale Veröffentlichungsnummer : **WO 91/10172 11.07.91 Gazette 91/15**

## (54) BISKATIONISCHE SÄUREAMID- UND -IMIDDERIVATE ALS LADUNGSSTEUERMITTEL.

(30) Priorität : **28.12.89 DE 3943048**
**05.09.90 DE 4028122**

(43) Veröffentlichungstag der Anmeldung : **07.10.92 Patentblatt 92/41**

(45) Bekanntmachung des Hinweises auf die Patenterteilung : **20.09.95 Patentblatt 95/38**

(84) Benannte Vertragsstaaten : **BE CH DE FR GB IT LI NL SE**

(56) Entgegenhaltungen :
**EP-A- 0 087 988**
**EP-A- 0 154 740**
**EP-A- 0 156 494**
**EP-A- 0 168 224**

(73) Patentinhaber : **HOECHST AKTIENGESELLSCHAFT**
**D-65926 Frankfurt am Main (DE)**

(72) Erfinder : **MACHOLDT, Hans-Tobias**
**Waldstrasse 20**
**D-6100 Darmstadt (DE)**
Erfinder : **SCHIESSLER, Siegfried**
**Rother Weingartenweg 48**
**D-6232 Bad Soden am Taunus (DE)**
Erfinder : **GITZEL, Jörg**
**Domherrnstrasse 2**
**D-6234 Hattersheim am Main (DE)**
Erfinder : **DIETZ, Erwin**
**St.-Matthäus-Strasse 7**
**D-6233 Kelkheim (Taunus) (DE)**

**EP 0 506 867 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Verwendung von biskationischen Säureamid- und -imidderivaten als farblose Ladungssteuermittel in Tonern und Entwicklern für elektrophotographische Aufzeichnungsverfahren.

Die erfindungsgemäßen Verbindungen besitzen durch die gezielte chemische Verknüpfung von speziellen Säureamid- bzw. -imidgruppierungen mit zwei jeweils ammonium- oder phosphoniumhaltigen Komponenten, wobei die Kombination dieser Bausteine über die jeweiligen Amid- bzw. imidstickstoffe erfolgt, besonders hohe und konstante Ladungssteuereigenschaften, ausgezeichnete Temperaturstabilitäten und sehr gute Dispergierbarkeit.

Bei elektrophotographischen Aufzeichnungsverfahren wird auf einem Photoleiter ein "latentes Ladungsbild" erzeugt. Dies erfolgt beispielsweise durch Aufladung eines Photoleiters durch eine Coronaentladung und anschließende bildmäßige Belichtung der elektrostatisch aufgeladenen Oberfläche des Photoleiters, wobei durch die Belichtung der Ladungsabfluß zur geerdeten Unterlage an den belichteten Stellen bewirkt wird. Anschließend wird das so erzeugte "latente Ladungsbild" durch Aufbringen eines Toners entwickelt. In einem darauffolgenden Schritt wird der Toner vom Photoleiter auf beispielsweise Papier, Textilien, Folien oder Kunststoff übertragen und dort beispielsweise durch Druck, Strahlung, Hitze oder Lösungsmitteleinwirkung fixiert. Der benutzte Photoleiter wird anschließend gereinigt und steht für einen neuen Aufzeichnungsvorgang zur Verfügung.

In zahlreichen Patentschriften wird die Optimierung von Tonern beschrieben, wobei u.a. der Einfluß des Tonerbindemittels (Variation von Harz/ Harzkomponenten oder Wachs/Wachskomponenten), der Einfluß von Steuermitteln oder anderen Zusatzsstoffen oder der Einfluß von Carriern (bei Zweikomponentenentwicklern) und Magnetpigmenten (bei Einkomponentenentwicklern) untersucht werden.

Ein Maß für die Tonerqualität ist seine spezifische Aufladung q/m (Ladung pro Maßeinheit). Neben Vorzeichen und Höhe der elektrostatischen Aufladung ist vor allem das schnelle Erreichen der gewünschten Ladungshöhe und die Konstanz dieser Ladung über einen längeren Aktivierzeitraum hinweg ein entscheidendes Qualitätskriterium. In der Praxis ist dies insofern von zentraler Bedeutung, als der Toner im Entwicklungsgemisch, bevor er auf den Photoleiter übertragen wird, einer erheblichen Aktivierzeit ausgesetzt sein kann, da er teilweise für einen Zeitraum der Herstellung von bis zu mehreren tausend Kopien im Entwicklergemisch verbleibt. Darüberhinaus ist die Unempfindlichkeit des Toners gegen Klimaeinflüsse, wie Temperatur und Luftfeuchtigkeit, ein weiteres wichtiges Eignungskriterium.

Sowohl positiv als auch negativ aufladbare Toner finden Verwendung in Kopierern und Laserdruckern in Abhängigkeit vom Verfahrens- und Gerätetyp.

Um elektrophotographische Toner oder Entwickler mit entweder positiver oder negativer triboelektrischer Aufladung zu erhalten, werden häufig sogenannte Ladungssteuermittel (auch Ladungskontrollmittel genannt) zugesetzt. Dabei ist neben dem Vorzeichen der Ladungssteuerung das Ausmaß des Steuereffektes von Bedeutung, da eine höhere Wirksamkeit eine geringe Einsatzmenge erlaubt.

Da Tonerbindemittel allein in der Regel eine starke Abhängigkeit der Aufladung von der Aktivierzeit aufweisen, ist es Aufgabe eines Ladungssteuermittels, zum einen Vorzeichen und Höhe der Toneraufladung einzustellen und zum anderen der Aufladungsdrift des Tonerbindemittels entgegenzuwirken und für Konstanz der Toneraufladung zu sorgen.

Ladungssteuerungsmittel, die nicht verhindern können, daß der Toner bzw. Entwickler bei längerer Gebrauchsdauer eine hohe Ladungsdrift zeigt (Alterung), die sogar bewirken kann, daß der Toner bzw. Entwickler eine Ladungsumkehr erfährt, sind daher für die Praxis ungeeignet.

Vollfarbkopierer und -laserdrucker arbeiten nach dem Prinzip der Trichromie, welches eine exakte Farbtonabstimmung der drei Grundfarben (Gelb, Cyan und Magenta) erforderlich macht. Geringste Farbtonverschiebungen auch nur eines der drei Grundfarben verlangt zwingend eine Farbtonverschiebung der beiden anderen Farben, um auch dann originaltreue Vollfarbkopien bzw. -drucke produzieren zu können.

Wegen dieser in Farbtonern erforderlichen präzisen Abstimmung der Coloristik der einzelnen Farbmittel aufeinander, sind Ladungssteuermittel absolut ohne Eigenfarbe ganz besonders wichtig.

Bei Farbtonern müssen die drei Toner Gelb, Cyan und Magenta neben den genau definierten farblichen Anforderungen auch hinsichtlich ihrer triboelektrischen Eigenschaften exakt aufeinander abgestimmt sein. Diese triboelektrische Abstimmung ist erforderlich, weil beim Vollfarbdruck bzw. bei der Vollfarbkopie aufeinanderfolgend die drei Farbtoner (bzw. vier Farbtoner, wenn Schwarz miteinbezogen wird) im selben Gerät übertragen werden müssen.

Von Farbmitteln ist bekannt, daß sie die triboelektrische Aufladung von Tonern teilweise sehr nachhaltig beeinflussen können (H.-T. Macholdt, A. Sieber, Dyes & Pigments 9 (1988), 119-27, US-PS 4057426). Wegen der unterschiedlichen triboelektrischen Effekte von Farbmitteln und des daraus resultierenden teilweise sehr

ausgeprägten Einflusses auf die Toneraufladbarkeit ist es nicht möglich, sie in eine einmal erstellte Tonerbasisrezeptur als ausschließliches Farbmittel hinzuzufügen. Vielmehr kann es notwendig werden, für jedes Farbmittel eine eigene Rezeptur zu erstellen, für welches z.B. Art und Menge des benötigten Ladungssteuermittels speziell zugeschnitten werden. Dieses Vorgehen ist entsprechend aufwendig und kommt bei Farbtonern für Prozeßfarbe (Trichromie) noch zusätzlich zu den bereits beschriebenen Schwierigkeiten hinzu.

Daher sind hochwirksame farblose Ladungssteuermittel erforderlich, die imstande sind, das unterschiedliche triboelektrische Verhalten verschiedener Farbmittel zu kompensieren und dem Toner die gewünschte Aufladung zu verleihen. Auf diese Art und Weise können triboelektrisch sehr unterschiedliche Farbmittel anhand einer einmal erstellten Tonerbasisrezeptur mit ein und demselben Ladungssteuermittel in den verschiedenen erforderlichen Tonern (Gelb, Cyan, Magenta und gegebenenfalls Schwarz) eingesetzt werden. Darüberhinaus ist für die Praxis wichtig, daß die Ladungssteuermittel eine hohe Thermostabilität und eine gute Dispergierbarkeit besitzen. Typische Einarbeitungstemperaturen für Ladungssteuermittel in die Tonerharze liegen bei Verwendung von z.B. Knetern oder Extrudern zwischen 100°C und 200°C. Dementsprechend ist eine Thermostabilität von 200°C, besser noch 250°C, von großem Vorteil. Wichtig ist auch, daß die Thermostabilität über einen längeren Zeitraum (ca. 30 min.) und in verschiedenen Bindemittelsystemen gewährleistet ist. Typische Tonerbindemittel sind Polymerisations-, Polyadditions- und Polykondensationsharze wie beispielsweise Styrol-, Styrolacrylat-, Styrolbutadien-, Acrylat-, Polyester-, Amid-, Amin-, Ammonium-, Ethylen-, Phenol- und Epoxidharze, einzeln oder in Kombination, die noch weitere Inhaltstoffe, wie Farbmittel, Wachse oder Fließhilfsmittel, enthalten können bzw. im Nachhinein zugesetzt bekommen können.

Dies ist wichtig, da immer wieder auftretende Matrixeffekte zum frühzeitigen Zersetzen des Ladungssteuermittel im Tonerharz führen, wodurch eine dunkelgelbe oder dunkelbraune Färbung des Tonerharzes erfolgt und der Ladungssteuereffekt ganz oder teilweise verloren geht.

Für eine gute Dispergierbarkeit ist es von großem Vorteil, wenn das Ladungssteuermittel möglichst keine wachsartigen Eigenschaften, keine Klebrigkeit und einen Schmelz- bzw. Erweichungspunkt von > 150°C besser > 200°C aufweist. Eine Klebrigkeit führt häufig zu Problemen beim Zudosieren in die Tonerformulierung, und niedrige Schmelz- bzw. Erweichungspunkte können dazu führen, daß beim Eindispergieren keine homogene Verteilung erreicht wird, da sich das Material z.B. tröpfchenförmig im Trägermaterial zusammenschließt.

Farblose Ladungssteuermittel werden in zahlreichen Patentschriften beansprucht. So beschreiben beispielsweise DE-OS 3144017, US-PS 4656112, JP-OS 61-236557 Metallkomplexe und Metallorganyle, DE-OS 3837345, DE-OS 3738948, DE-OS 3604827, EP-OS 242420, EP-OS 203532, US-PS 4684596, US-PS 4683188, US-PS 4493883 Ammonium- und Immoniumverbindungen und DE-OS 3912396, US-PS 3893939, US-PS 4496643 Phosphoniumverbindungen als farblose Ladungssteuermittel für elektrophotographische Toner.

Allerdings weisen die bisher bekannten farblosen Ladungssteuermittel eine Reihe von Nachteilen auf, die den Einsatz in der Praxis stark einschränken, bzw. zum Teil unmöglich machen.

So haben die in DE-OS 3144017 und US-PS 4656112 beschriebenen Chrom-, Eisen-, Koblat- und Zinkkomplexe und die in JP-OS 61-236557 beschriebenen Antimonorganyle neben der Schwermetallproblematik auch den Nachteil, daß sie teilweise nicht wirklich farblos sind, und somit in Farbtonern nur eingeschränkt Anwendung finden können.

Die bekannten, an sich geeigneten, quaternären Ammoniumverbindungen sind häufig schwierig zu dispergieren, was zu einer ungleichmäßigen Aufladung des Toners führt. Zudem tritt oft das Problem auf, daß die von diesen Verbindungen erzeugte Tonerladung nicht über einen längeren Aktivierzeitraum (bis zu 24 Stunden Aktivierdauer) hinweg stabil ist, insbesondere bei hoher Temperatur und Luftfeuchtigkeit (EP-OS 242420), was dann im Verlaufe eines Kopier- oder Druckprozesses zur Anreicherung falsch bzw. nicht genügend aufgeladener Tonerteilchen führt und damit den Prozeß zum Erliegen bringt. Ferner ist bekannt, daß Ladungssteuermittel auf Ammonium- bzw. Immoniumbasis empfindlich gegen Licht oder mechanische Einwirkungen (EP-OS 203532, US-PS 4683188) und thermisch labil sein können, und daß sie Zersetzungsprodukte bilden, die sich nachteilig auf die triboelektrische Aufladung des Toners auswirken können (US-PS 4684596) und/oder eine starke, oft dunkelbraune, Eigenfarbe aufweisen (DE-OS 3738948, DE-OS 3604827, US-PS 4493883). Darüberhinaus zeigen sie oft wachsartiges Verhalten, zum Teil Wasserlöslichkeit und/oder geringe Wirksamkeit als Ladungssteuermittel.

An sich geeignete Ladungssteuermittel auf Basis hochgradig fluorierter Ammonium-, Immonium- und Phosphoniumverbindungen (DE-OS 3912396, DE-OS 3837345) haben den Nachteil einer aufwendigen Synthese, wodurch hohe Herstellungskosten für die entsprechenden Substanzen anfallen, und sind nicht ausreichend thermostabil.

Phosphoniumsalze sind als Ladungssteuermittel weniger wirksam als Ammoniumsalze (US-PS 3893939, US-PS 4496643) und können toxikologisch problematisch sein.

Außer in elektrophotographischen Tonern und Entwicklern können Ladungssteuermittel auch zur Verbesserung der triboelektrischen Aufladung von Pulvern und Lacken, insbesondere in triboelektrisch bzw. elektrokinetisch versprühten Pulverlacken, wie sie zur Oberflächenbeschichtung von Gegenständen aus beispielsweise Metall, Holz, Kunststoff, Glas, Keramik, Beton, Textilmaterial, Papier oder Kautschuk zur Anwendung kommen, eingesetzt werden. Die Pulverlacktechnologie kommt u.a. beim Lackieren von Kleingegenständen, wie Gartenmöbeln, Campingartikeln, Haushaltsgeräten, Fahrzeugkleinteilen, Kühlschränken und Regalen, sowie beim Lackieren von kompliziert geformten Werkstücken zur Anwendung. Der Pulverlack bzw. das Pulver enthält seine elektrostatische Ladung im allgemeinen nach einem der beiden folgenden Verfahren:

a) Beim Corona-Verfahren wird der Pulverlack bzw. das Pulver an einer geladenen Corona vorbeigeführt und hierbei aufgeladen.

b) Beim triboelektrischen bzw. elektrokinetischen Verfahren wird vom Prinzip der Reibungselektrizität Gebrauch gemacht. Der Pulverlack bzw. das Pulver erhält im Sprühgerät eine elektrostatische Aufladung, die der Ladung des Reibungspartners, im allgemeinen ein Schlauch oder Sprührohr (beispielsweise aus Polytetrafluorethylen), entgegengesetzt ist.

Auch eine Kombination von beiden Verfahren ist möglich.

Als Pulverlackharze werden typischerweise Epoxidharze, carboxyl- und hydroxylgruppenhaltige Polyesterharze und Acrylharze zusammen mit den entsprechenden Härtern eingesetzt. Auch Kombinationen von Harzen finden Verwendung. So werden beispielsweise häufig Epoxidharze in Kombination mit carboxyl- und hydroxylgruppenhaltigen Polyesterharzen eingesetzt.

Typische Härterkomponenten für Epoxidharze sind beispielsweise Säureanhydride, Imidazole sowie Dicyandiamid und deren Abkömmlinge. Für hydroxylgruppenhaltige Polyesterharze sind typische Härterkomponenten beispielsweise Säureanhydride, verkappte Isocyanate, Bisacylurethane, Phenolharze, Melaminharze, für carboxylgruppenhaltige Polyesterharze sind typische Härterkomponenten beispielsweise Triglycidylisocyanurate oder Epoxidharze. In Acrylharzen kommen als typische Härterkomponenten beispielsweise Oxazoline, Isocyanate, Triglycidylisocyanurate oder Dicarbonsäure als Härterkomponenten zur Anwendung.

Der Mangel ungenügender Aufladung ist vor allem bei triboelektrisch bzw. elektrokinetisch versprühten Pulvern und Pulverlacken, die auf Basis von Polyesterharzen, insbesondere carboxylgruppenhaltigen Polyestern, oder auf Basis von sogenannten Mischpulvern auch Hybridpulver genannt, hergestellt worden sind, zu beobachten. Unter Mischpulvern versteht man Pulverlacke, deren Harzbasis aus einer Kombination von Epoxidharz und carboxylgruppenhaltigem Polyesterharz besteht. Die Mischpulver bilden die Basis für die in der Praxis am häufigsten vertretenen Pulverlacke.

Ziel der vorliegenden Erfindung war es daher, verbesserte, besonders wirksame farblose Ladungssteuermittel zu finden, wobei neben der Ladungshöhe das schnelle Erreichen und die Konstanz dieser Ladung gewährleistet sein mußte, und der Ladungseffekt nicht empfindlich gegenüber Temperatur- und Luftfeuchtigkeitsveränderungen sein durfte. Darüberhinaus sollten diese Verbindungen in hohem Maße thermostabil sein, vor allem auch über einen längeren Zeitraum hinweg im jeweiligen Trägermaterial (Harz), sowie wasserunlöslich, gut dispergierbar und verträglich mit den Tonerinhaltsstoffen. Des weiteren sollte die Synthese der Verbindungen wenig aufwendig und ihre Herstellung kostengünstig sein.

Überraschenderweise hat sich nun gezeigt, daß spezielle, biskationische Säureamid- und Säureimidderivate besonders wirksame Ladungssteuermittel für elektrophotographische Toner und Entwickler sind, und darüberhinaus auch als ladungsverbesserndes Mittel in Pulvern und Lacken zur Oberflächenbeschichtung, insbesondere elektrokinetisch versprühten Pulverlacken eingesetzt werden können.

Auf Grund ihrer Farblosigkeit, hohen Wirksamkeit, guten Verträglichkeit und Dispergierbarkeit in gängigen Tonerharzen und chemischen Inertheit, sowie wegen der Unempfindlichkeit des Ladungssteuereffektes gegenüber Temperatur- und Luftfeuchtigkeitschwankung sind die Verbindungen insbesondere für den Einsatz in Farbtonern bzw. -entwicklern für Vollfarbkopierer und -laserdrucker nach dem Prinzip der Trichromie (substraktive Farbmischung), aber auch für farbige Toner bzw. Entwickler im allgemeinen und für schwarze Toner oder Entwickler geeignet. Des weiteren eignen sich die Verbindungen auch für das Beschichten von Carriern.

Ein großer technischer Vorteil dieser gut dispergierbaren Verbindungen liegt darin, daß Substanzen derselben Verbindungsklasse je nach Kationen-Anionenkombination entweder als positives oder als negatives Steuermittel eingesetzt werden können. Somit werden Probleme bei der Einarbeitung in das Tonerbindemittel und der Verträglichkeit mit dem Tonerbindemittel nach Erstellung einer Tonerbasisrezeptur minimiert. Es können somit sowohl Positiv- wie auch Negativtoner anhand einer festen Tonerbasisrezeptur (bestehend aus Tonerbindemittelm, Farbmittel, Fließhilfsmittel und ggfs. weiteren Komponenten) durch Einarbeitung des gewünschten Steuermittels hergestellt werden.

Von ganz besonderem Vorteil ist, daß die Synthese der erfingungsgemäß beanspruchten Verbindungen wenig aufwendig und ihre Herstellung sehr kostengünstig ist.

Gegenstand der vorliegenden Erfindung ist somit die Verwendung von biskationischen Säureamid- und

-imidderivaten der allgemeinen Formel (I) und/ oder (II) und/oder (III)

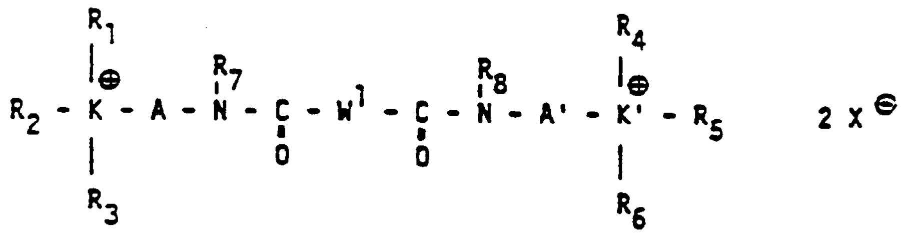

(I)

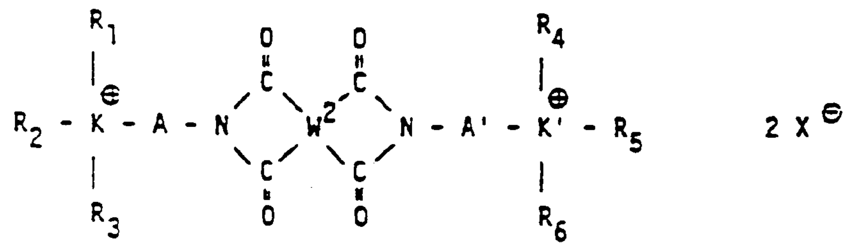

(II)

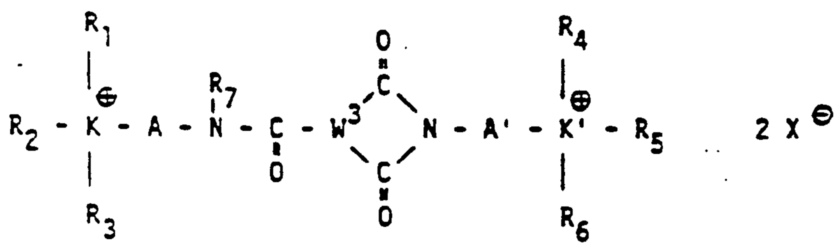

(III)

wobei $R_1$ bis $R_8$ unabhängig voneinander ein Wasserstoffatom, einen Kohlenwasserstoffrest, der durch Heteroatome unterbrochen sein kann, wie z.B. geradkettig oder verzweigte, gesättigte oder ungesättigte Alkylgruppen von 1 bis 30 C-Atomen, vorzugsweise von 1 bis 22 C-Atomen, Polyoxalkylengruppen bevorzugt Polyoxethylen- bzw. Polyoxpropylengruppen, der allgemeinen Formel -(Alkylen-O)n-R, worin R ein H-Atom oder eine Alkyl($C_1$-$C_4$)Gruppe, Acylgruppe, wie beispielsweise die Acetyl-, Benzoyl-, oder Naphthoylgruppe, und n eine Zahl von 1 bis 10 vorzugsweise von 1 bis 4 ist, ein- oder mehrkernige cycloaliphatische Reste von 5 bis 12 C-Atomen wie beispielsweise Cyclohexyl- oder Cyclopentylgruppen, ein- oder mehrkernige aromatische Reste wie beispielsweise Phenyl-, Naphthyl-, Tolyl-, oder Biphenylreste oder araliphatische Reste wie beispielsweise den Benzylrest, darstellen, wobei die aliphatischen, cycloaliphatischen, araliphatischen und aromatischen Reste durch Säuregruppen, bevorzugt Carbonsäure und/oder Sulfonsäuregruppen, bzw. deren Salze, Amide oder Ester, Alkyl($C_1$-$C_4$)-, Hydroxy-, Alkoxy($C_1$-$C_4$)-gruppen, primäre, sekundäre oder tertiäre Aminogruppen, wie beispielsweise N-Monoalkyl($C_1$-$C_4$)amino- oder N-Dialkyl ($C_1$-$C_4$)-aminogruppen, sowie durch Fluor-, Chlor- oder Bromatome, die aliphatischen Reste bevorzugt durch 1 bis 45 Fluoratome substituiert sein können, und wobei die aliphatischen, cycloaliphatischen, araliphatischen oder aromatischen Ringsysteme ein oder mehrere Heteroatome wie beispielsweise Stickstoff- und/oder Sauerstoff- und/oder Schwefel- und/oder Phosphoratome enthalten können, und wobei unabhängig voneinander $R_1$ und $R_2$ in Verbindung mit K bzw. $R_4$ und $R_5$ in Verbindung mit K′ sich zu einem gesättigten oder ungesättigten, bevorzugt aromatischem Ringsystem mit 5 bis 7 Atomen zusammenschließen können, das weitere Heteroatome, bevorzugt Stickstoff- und/oder Sauerstoff- und/oder Schwefelatome enthalten kann, wobei das jeweilige Ringsystem wiederum substituiert und/oder durch Ankondensation von bzw. Verbrückung zu weiteren Ringsystemen modifiziert sein kann, und wobei für den Fall daß $R_1$ oder $R_2$ bzw. $R_4$ oder $R_5$ eine Doppelbindung zu K oder K′ ausbilden, $R_3$ oder $R_5$ gegenstandslos werden, sind, und/oder einer der Reste $R_1$, $R_2$ oder $R_3$ sich mit $R_7$ bzw. einer der Reste $R_4$, $R_5$ oder $R_6$ sich mit $R_5$ zu einer aliphatischen Brücke aus 2 bis 5 Kohlenstoffatomen zusammenschließen kann, und wobei A, A′ organische Brückenglieder, vorzugsweise unabhängig voneinander geradkettig oder

verzweigte, gesättigte oder ungesättigte Alkylenglieder von 1 bis 30 Kohlenstoffatomen, vorzugsweise 1 bis 12 Kohlenstoffatomen, ein- oder mehrkernige cycloaliphatische Glieder wie beispielsweise Cyclohexylen oder Cyclopentylen, ein- oder mehrkernige aromatische Glieder wie beispielsweise Phenylen, Naphthylen, Tolylen, Biphenylen oder araliphatische Glieder wie beispielsweise Benzylen, Xylylen, Mesitylen, Benzoylen oder Benzoylenamid, darstellen, wobei die aliphatischen, cycloaliphatischen, araliphatischen und aromatischen Glieder durch Säuregruppen, bevorzugt Carbonsäuren und/oder Sulfonsäuregruppen, bzw. deren Salze, Amide, Alkyl($C_1$ $C_4$)-, Hydroxy-, Alkoxy($C_1$-$C_4$)Gruppen, primäre, sekundäre oder tertiäre Aminogruppen, wie beispielsweise N-Monoalkyl($C_1$-$C_4$)amino- oder N-Dialkyl($C_1$-$C_4$)aminogruppen, sowie durch Fluor-, Chlor- oder Bromatome, die aliphatische Glieder bevorzugt durch 1 bis 45 Fluoratome, substituiert sein können, und wobei die aliphatischen, aromatischen und araliphatischen Ringsysteme ein oder mehrere Heteroatome, wie beispielsweise Stickstoff- und/oder Sauerstoff- und/oder Schwefel- und/oder Phosphoratome enthalten können, und wobei $W^1$, $W^2$, $W^3$ unabhängig voneinander ein organisches Brückenglied, wie beispielsweise ein geradkettiges oder verzweigtes, gesättigtes oder ungesättigtes aliphatisches Brückenglied mit 1 bis 30 Kohlenstoffatomen, bevorzugt 1 bis 22 Kohlenstoffatomen, ein Polyoxalkylenglied, bevorzugt ein Polyoxethylen- oder Polyoxpropylenglied der allgemeinen Formel $-CH_2-O-(Alkylen[C_1-C_5]-O)m-CH_2-$, wobei m eine Zahl von 0 bis 10 vorzugsweise von 1 bis 4 ist, ein ein- oder mehrkerniges cycloaliphatisches Brückenglied mit 5 bis 12 C-Atomen wie beispielsweise Cyclopentylen oder Cyclohexylen, ein ein- oder mehrkerniges aromatisches Brückenglied wie beispielsweise Phenylen, Naphthylen, Tolylen oder Biphenylen, ein araliphatisches Brückenglied, wie beispielsweise Benzylen darstellt, wobei die aliphatischen, cycloaliphatischen, araliphatischen und aromatischen Glieder durch Säuregruppen, bevorzugt Carbonsäuren und/oder Sulfonsäuregruppen bzw. deren Salze, Amide oder Ester, Hydroxy-, Alkyl($C_1$-$C_4$)-, Alkoxy($C_1$-$C_4$)-gruppen, primäre, sekundäre oder tertiäre Aminogruppen, wie beispielsweise N-Monoalkyl($C_1$-$C_4$)amino- oder N-Dialkylamino($C_1$-$C_4$)-gruppen, sowie durch Fluor-, Chlor- oder Bromatome, die aliphatischen Glieder bevorzugt durch 1 bis 45 Fluoratome substituiert sein können, und wobei die aliphatischen Zwischenglieder sowie die cycloaliphatischen, die araliphatischen und die aromatischen Ringsysteme ein oder mehrere Heteroatome wie beispielsweise Stickstoff-, und/oder Sauerstoff- und/oder Schwefel-, und/oder Phosphoratome, enthalten können, wobei $W^1$ ein zweiwertiges, $W^2$ ein vierwertiges und $W^3$ ein dreiwertiges Zwischenglied ist und wobei $W^1$ im Falle der allgemeinen Formel (I) auch eine direkte Bindung bedeuten kann,
und K bzw. K′ unabhängig voneinander ein Stickstoff-, Phosphor-, Arsen- oder Antimon, bevorzugt ein Stickstoffatom ist,
und das Anion $X^{\ominus}$ das stöchiometrische Äquivalent eines oder mehrerer gemischter oder nichtgemischter Anionen ist, wobei die Verbindung auch als Mischkristall mit verschiedenen Kationen der allgemeinen Formel (I) bis (III) vorliegen kann, einzeln oder in Kombination als Ladungssteuermittel in elektrophotographischen Tonern und Entwicklern, die zum elektrophotographischen Kopieren bzw. Vervielfältigen von Vorlagen sowie zum Drucken von elektronisch, optisch oder magnetisch gespeicherten Informationen oder im Colorproofing eingesetzt werden.

Darüberhinaus sind die erfindungsgemäß beanspruchten Verbindungen geeignet als Ladungssteuermittel in Pulvern und Lacken zur Oberflächenbeschichtung von Gegenständen aus Metall, Holz, Kunststoff Glas, Keramik, Beton, Textilmaterial, Papier oder Kautschuk, insbesondere in triboelektrisch bzw. elektrokinetisch versprühten Pulverlacken. Die erfindungsgemäß beanspruchten Verbindungen liegen in einer Menge von etwa 0,01 bis etwa 30 Gewichtsprozent, vorzugsweise 0,1 bis etwa 5 Gewichtsprozent, homogen verteilt in dem jeweiligen Toner, Entwickler, Lack oder Pulver vor. Außerdem können diese Verbindungen auch als ladungsverbesserndes Mittel in die Beschichtung von Carriern, die in Entwicklern von elektrophotographischen Kopierern oder Druckern eingesetzt werden, eingearbeitet werden.

Besonders geeignet sind Verbindungen, in denen K und K′ Stickstoff bedeuten, $R_1$ bis $R_6$ unabhängig voneinander H-Atome, geradkettig oder verzweigte Alkylgruppen ($C_1$-$C_6$), wie beispielsweise Methyl, Ethyl, n-Propyl, iso-Propyl, tertiär-Butyl, Pentyl und/oder Hexyl bedeuten, und solche in denen unabhängig voneinander $R_1$ und $R_2$ bzw. $R_4$ und $R_5$ unter Einbeziehung von K bzw. K′, sich zu einem gesättigten oder ungesättigten, heterocyclischen Ringsystem mit einem oder mehreren Stickstoffatomen als Heteroatom wie beispielsweise Pyrrol, Pyrrolin, Pyrrolidin, Pyrazol, Pyrazolon, Pyrazolin, Hexamethylenimin, Imidazol, Oxazol, Thiazol, Triazol, Pyridin, Piperidin, Pyrazin, Piperazin, Pyrimidin, Morpholin zusammenschließen können, wobei diese Ringsysteme wiederum subtituiert sein können, bevorzugt mit geradkettigen Alkyl($C_1$-$C_4$)-gruppen und/oder durch Ankondensation bzw. Verbrückung sich zu Ringsystemen wie Chinolin, Indol, Indolin, Purin, Chinoxalin, Benzthiazol, Acridin, Benzchinolin, Carbazol, Benzophenazin, Phenanthrolin, Bipiperidin, Bipyridin, Phenazin, Benzacridin oder Nicotin vergrößern können, wobei $R_3$ und/oder $R_6$ ein Wasserstoffatom oder eine Alkyl($C_1$-$C_4$)gruppe darstellen, wobei $R_3$ und/oder $R_6$ auch ganz entfallen können, falls in dem unter Einbeziehung von K bzw. K′ ausgebildeten Ringsystem eine Doppelbindung zwischen K bzw. K′ und einem Nachbaratom besteht, $R_7$ und $R_8$ ein Wasserstoffatom bedeuten,

und A und A' unabhängig voneinander ein $-CH=C(COOH)-CH_2-$, $-CH_2-(CH_2-CH=CH-)n$ oder $-(CH_2-)n$ Brückenglied mit n=1 bis 12, bevorzugt 1 bis 4, ein Phenylen-, Naphthylen- oder

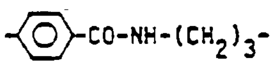

Brückenglied bedeutet,
$W^1$ ein Phenylen-, Naphtylen-, Cyclohexylen-, $(CH_2)q$, mit q=1 bis 12, oder ein $(CH_2-O(CH_2-CH_2-O)_r-CH_2-$ Brückenglied mit r=1 bis 4,
$W^2$ ein Phenylen- oder Naphthylen-Brückenglied, wobei die zur Imidbildung benötigten Carboxylgruppen im Falle des Phenylens jeweils in ortho-Stellung im Falle des Naphthylens in ortho- und/oder peri-Stellung zueinander stehen, oder ein Ethylendiamintetramethylen-Brückenglied,
und $W^3$ ein Phenylen- oder Naphthylenbrückenglied ist, wobei mindestens zwei an der Imidbindung beteiligten Carboxylgruppen sich in ortho- oder peri-Stellung zueinander befinden, und die dritte Carboxylgruppe sich im beliebiger Stellung dazu befinden kann,
wobei im Falle von A, A' bzw. $W^1$ die Phenylenbrückenglieder in 1,2; 1,3; 1,4-Stellung, bevorzugt in 1,3 und 1,4-Stellung, die Naphthylenbrückenglieder in 1,2 bis 1,8 sowie in 2,3 bis 2,8-Stellung und die Cyclohexylenbrückenglieder in 1,2; 1,3; 1,4-Stellung, bevorzugt in 1,3 bzw. 1,4-Stellung zu dem jeweiligen K oder K' auf der einen Seite und Imid- oder Amidstickstoff auf der anderen Seite verbrückt sind bzw. von den jeweiligen Carboxylfunktionen substuiert sind, und das Anion $X^{\ominus}$ das oder die Equivalente eines entsprechenden organischen oder anorganischen Anions bedeutet, an einwertigen anorganischen Anionen beispielsweise $BF_4^{\ominus}$, B $(Aryl)_4{}^{\ominus}$, wie z.B. Tetraphenylborat, Chlortetraphenylborat, Methyltetraphenylborat, Tetranaphthylborat, Tetrafluorphenylborat, Tetramethoxyphenylborat, Tetrabiphenylborat, Tetrabenzylborat, Tetrapyridylborat, $PF_6{}^{\ominus}$, $SCN^{\ominus}$, $HSO_4{}^{\ominus}$, $F^{\ominus}$, $Cl^{\ominus}$, $Br^{\ominus}$, $J^{\ominus}$, $CN^{\ominus}$, $ClO_4{}^{\ominus}$, Sulfat, Hydrogensulfat, Zinktetracyanat, Zinktetrathiocyanat, Wolframat, Molybdat, Phosphormolybdat und- wolframat sowie Silicomolybdat- und wolframat und an organischen Anionen beispielsweise Ethyl- und Methylsulfat, gesättigtes oder ungesättigtes aliphatisches oder aromatisches Carboxylat oder Sulfonat, wie beispielsweise Acetat, Lactat, Oxalat Benzoat, Salicylat, 2-Hydroxy-3-naphthoat, 2-Hydroxy-6-naphthoat, Ethylsulfonat, Phenylsulfonat, 4-Toluolsulfonat, 4-Aminotoluol-3-sulfonat, ferner perfluoriertes, gesättigtes oder ungesättigtes, aliphatisches oder aromatisches Carboxylat oder Sulfonat, wie beispielsweise Perfluoracetat, Perflouralkylbenzoat, Perfluorethylsulfonat oder Perfluoralkylbenzolsulfonat, sowie gesättigte und ungesättigte aliphatische oder aromatische Di- und Tricarbonsäure bzw. Di- und Trisulfonsäureanionen bedeuten wobei ganz besonders die Anionen $BF_4{}^{\ominus}$, B $(Aryl)_4{}^{\ominus}$, $PF_6{}^{\ominus}$, $SCN^{\ominus}$, $CH_3SO_4{}^{\ominus}$, $C_2H_5SO_4{}^{\ominus}$, $HSO_4{}^{\ominus}$ und

$$P\left[Mo_3\ O_{10}\right]_4^{3-}$$

geeignet sind.

An Einzelverbindungen seien beispielsweise aufgeführt:

| Verbindung | Kation | Anion |
|---|---|---|
| 1.1a | 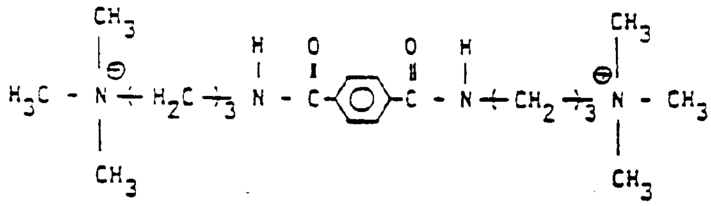 | 2x $BF_4^{\ominus}$ |
| 1.1b | s.o. | 2x $PF_6^{\ominus}$ |
| 1.1c | s.o. | 2x B 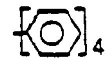 ⊖ |
| 1.1d | s.o. | 2x $SCN^{\ominus}$ |
| 1.1e | s.o. | 2x $CH_3SO_4^{\ominus}$ |
| 1.2a | 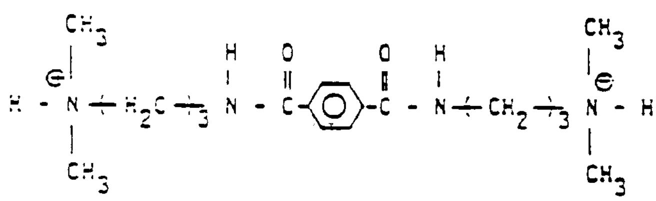 | 2x $BF_4^{\ominus}$ |
| 1.2b | s.o. | 2x $PF_6^{\ominus}$ |
| 1.2c | s.o. | 2x B 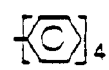 ⊖ |
| 1.2d | s.o. | 2x $SCN^{\ominus}$ |
| 1.2e | s.o. | 2x $CH_3SO_4^{\ominus}$ |
| 1.3a | 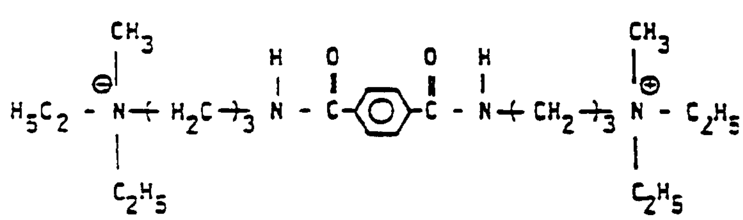 | 2x $BF_4^{\ominus}$ |
| 1.3b | s.o. | 2x B 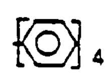 ⊖ |

| Verbindung | Kation | Anion |
|---|---|---|
| 1.4a | 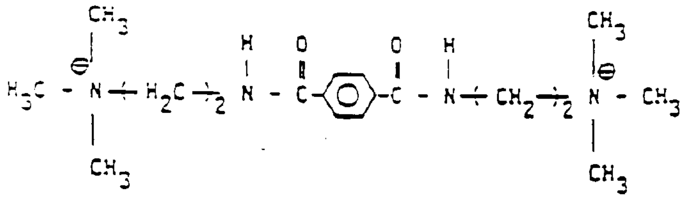 | 2x $BF_4^{\ominus}$ |
| 1.4b | s.o. | 2x 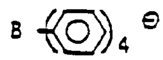 |
| 1.5a | 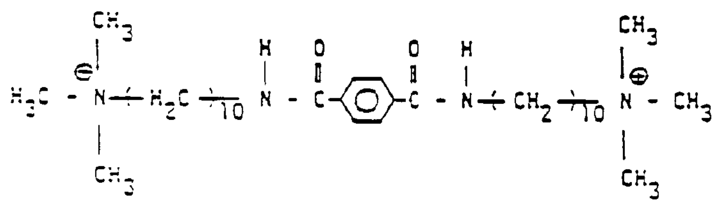 | 2x $BF_4^{\ominus}$ |
| 1.5b | s.o. | 2x 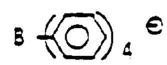 |
| 1.6a | 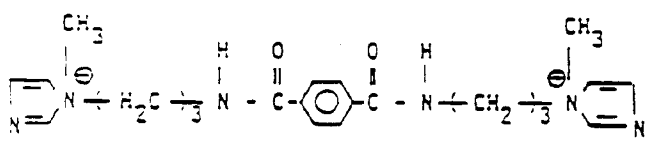 | 2x $BF_4^{\ominus}$ |
| 1.6b | s.o. | 2x 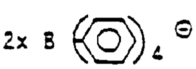 |
| 1.7a | 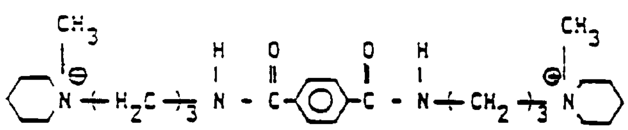 | 2x $BF_4^{\ominus}$ |
| 1.7b | s.o. | 2x 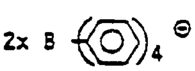 |

| Verbindung | Kation | Anion |
|---|---|---|
| 1.8a | 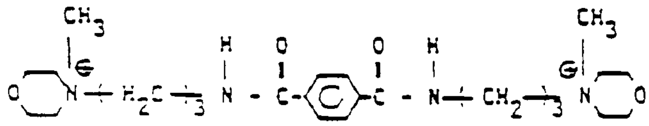 | 2x $BF_4^{\ominus}$ |
| 1.8b | s.o. | 2x B 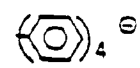 |
| 1.9a | 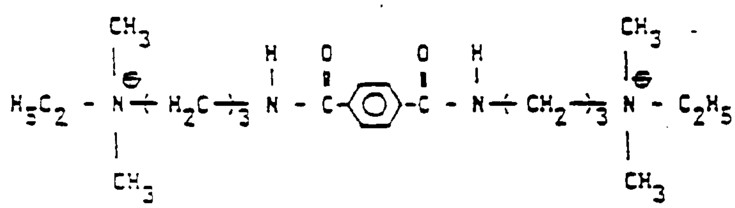 | 2x $BF_4^{\ominus}$ |
| 1.9b | s.o. | 2x B 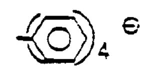 |
| 1.10a | 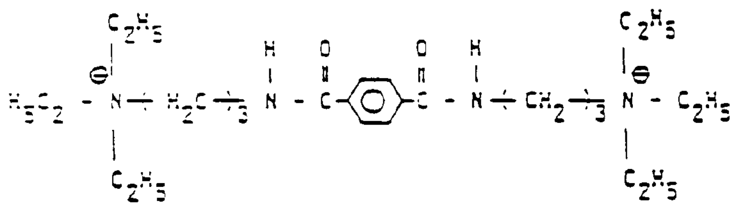 | 2x $BF_4^{\ominus}$ |
| 1.10b | s.o. | 2x B 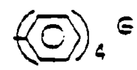 |
| 1.11a | 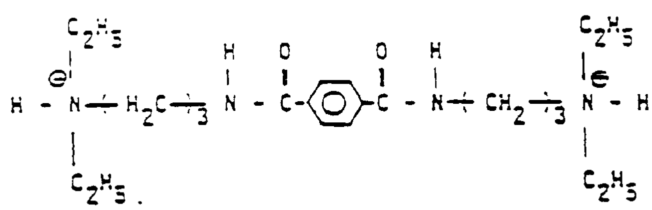 | 2x $BF_4^{\ominus}$ |
| 1.11b | s.o. | 2x $PF_6^{\ominus}$ |
| 1.11c | s.o. | 2x B 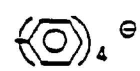 |
| 1.11d | s.o. | 2x $SCN^{\ominus}$ |
| 1.11e | s.o. | 2x $CH_3SO_4^{\ominus}$ |

| Verbindung | Kation | Anion |
|---|---|---|
| 2.1a | 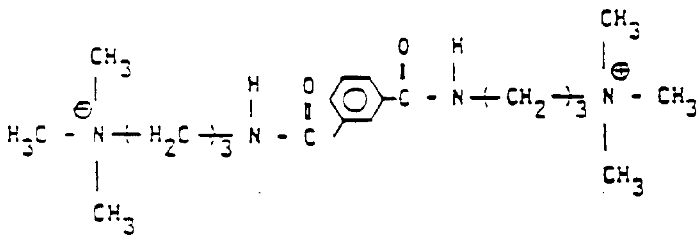 | 2x $BF_4^{\ominus}$ |
| 2.1b | s.o. | 2x 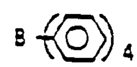 $^{\ominus}$ |
| 3.1a | cis/ trans 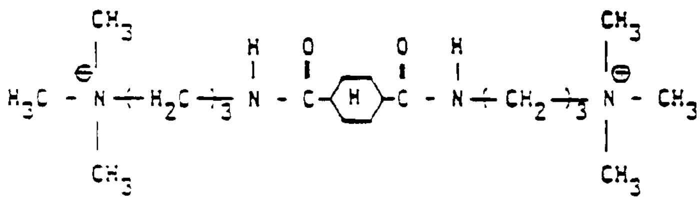 | 2x $BF_4^{\ominus}$ |
| 3.1b | s.o. | 2x $PF_6^{\ominus}$ |
| 3.1c | s.o. | 2x 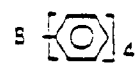 $^{\ominus}$ |
| 3.1d | s.o. | 2x $SCN^{\ominus}$ |
| 3.1e | s.o. | 2x $CH_3SO_4^{\ominus}$ |
| 3.2a | cis/ trans 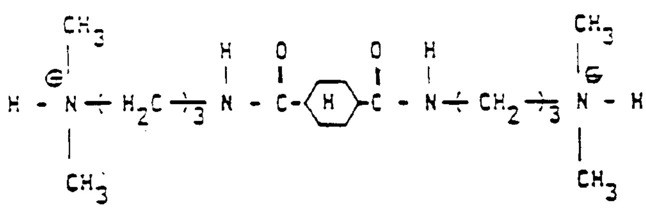 | 2x $BF_4^{\ominus}$ |
| 3.2b | s.o. | 2x $PF_6^{\ominus}$ |
| 3.2c | s.o. | 2x 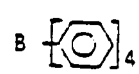 $^{\ominus}$ |
| 3.2d | s.o. | 2x $SCN^{\ominus}$ |
| 3.2e | s.o. | 2x $CH_3SO_4^{\ominus}$ |

| Verbindung | Kation | Anion |
|---|---|---|
| 3.3a cis/trans | 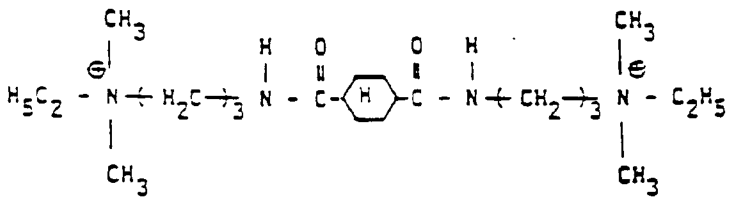 | 2x $BF_4^{\ominus}$ |
| 3.3b | s.o. | 2x B 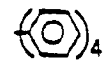 ⊖ |
| 3.4a cis/trans | 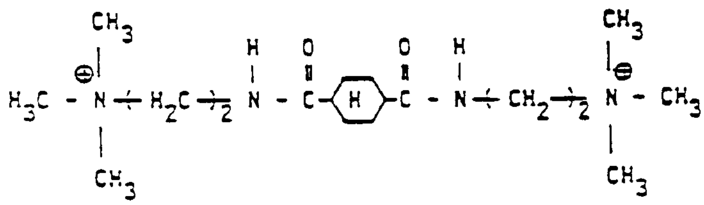 | 2x $BF_4^{\ominus}$ |
| 3.4b | s.o. | 2x B 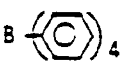 ⊖ |
| 3.5a cis/trans | 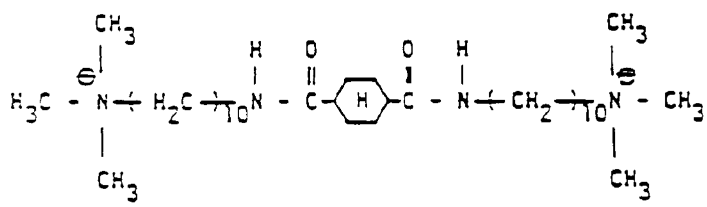 | 2x $BF_4^{\ominus}$ |
| 3.5b | s.o. | 2x B 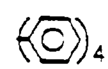 ⊖ |
| 4.1a | 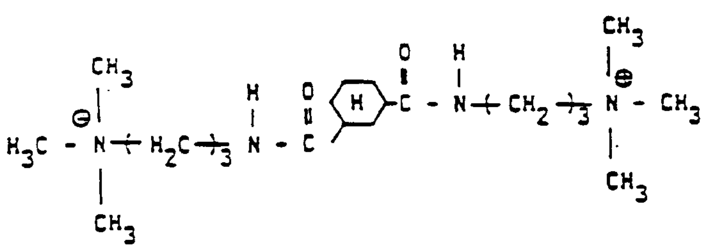 | 2x $BF_4^{\ominus}$ |
| 4.1b | s.o. | 2x B 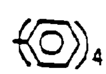 ⊖ |

| Verbindung | Kation | Anion |
|---|---|---|
| 5.1a | 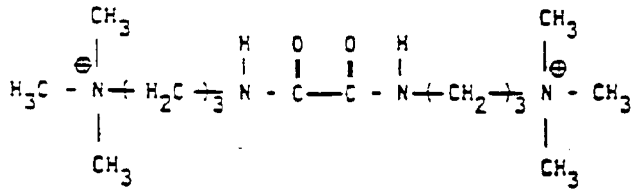 | 2x $BF_4^{\ominus}$ |
| 5.1b | s.o. | 2x B 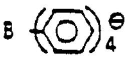 |
| 6.1a | 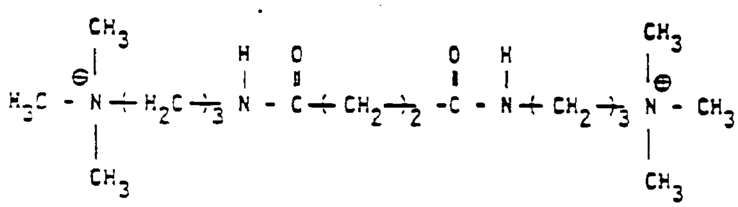 | 2x $BF_4^{\ominus}$ |
| 6.1b | s.o. | 2x $PF_6^{\ominus}$ |
| 6.1c | s.o. | 2x B 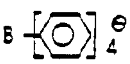 |
| 6.1d | s.o. | 2x $SCN^{\ominus}$ |
| 6.1e | s.o. | 2x $CH_3SO_4^{\ominus}$ |
| 6.2a | 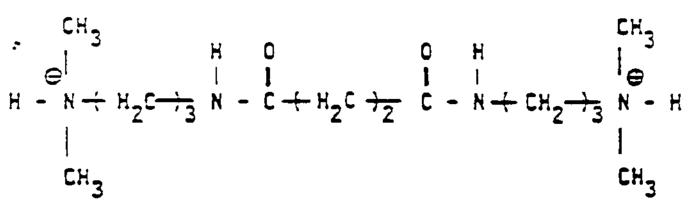 | 2x $BF_4^{\ominus}$ |
| 6.2b | s.o. | 2x $PF_6^{\ominus}$ |
| 6.2c | s.o. | 2x B 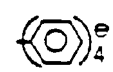 |
| 6.2d | s.o. | 2x $SCN^{\ominus}$ |
| 6.2e | s.o. | 2x $CH_3SO_4^{\ominus}$ |

| Verbindung | Kation | Anion |
|---|---|---|
| 6.3a | 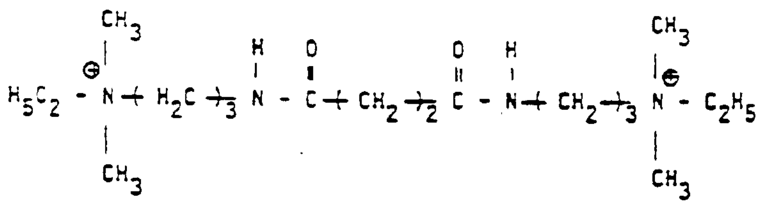 | 2x $BF_4^{\ominus}$ |
| 6.3b | s.o. | 2x B 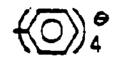 |
| 6.4a | 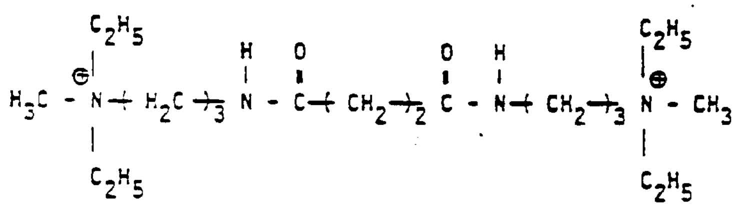 | 2x $BF_4^{\ominus}$ |
| 6.4b | s.o. | 2x $PF_6^{\ominus}$ |
| 6.4c | s.o. | 2x $SCN^{\ominus}$ |
| 6.4d | s.o. | 2x B 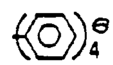 |
| 6.4e | s.o. | 2x $CH_3SO_4^{\ominus}$ |
| 6.5a | 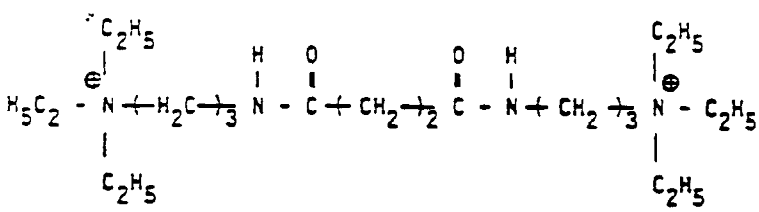 | 2x $BF_4^{\ominus}$ |
| 6.5b | s.o. | 2x B 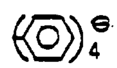 |

| Verbindung | Kation | Anion |
| --- | --- | --- |
| 7.1a | 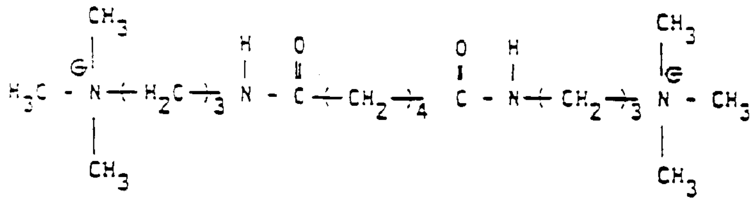 | 2x $BF_4^{\ominus}$ |
| 7.1b | s.o. | 2x 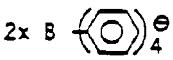 |
| 8.1a | 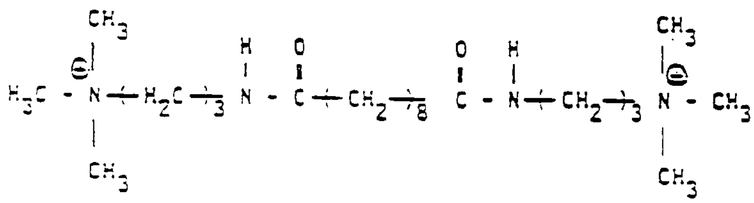 | 2x $BF_4^{\ominus}$ |
| 8.1b | s.o. | 2x 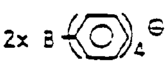 |
| 9.1a | 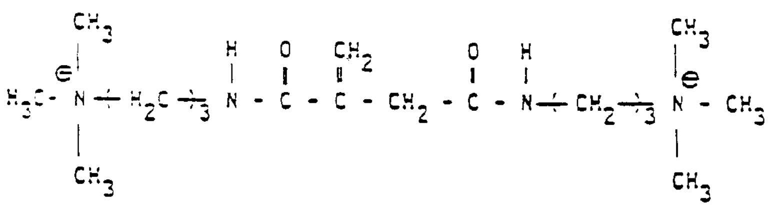 | 2x $BF_4^{\ominus}$ |
| 9.1b | s.o. | 2x 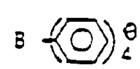 |
| 10.1a | 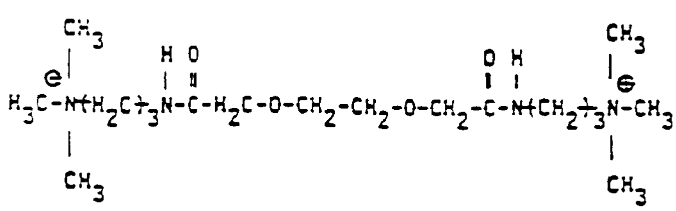 | 2x $BF_4^{\ominus}$ |
| 10.1b | s.o. | 2x 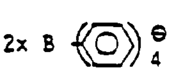 |

| Verbindung | Kation | Anion |
|---|---|---|
| 11.1a | 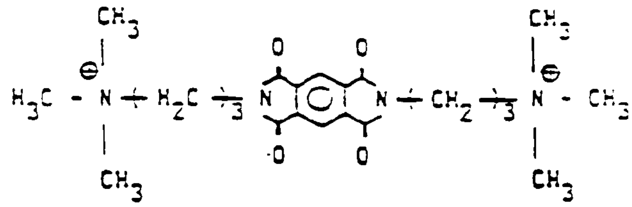 | 2x $BF_4^{\ominus}$ |
| 11.1b | s.o. | 2x $PF_6^{\ominus}$ |
| 11.1c | s.o. | 2x B 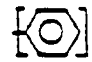 $_4^{\ominus}$ |
| 11.1d | s.o. | 2x $SCN^{\ominus}$ |
| 11.1e | s.o. | 2x $CH_3SO_4^{\ominus}$ |
| 11.1f | s.o. | 2/3x $P[Mo_3O_{10}]_4^{3\ominus}$ |
| 12.1a | 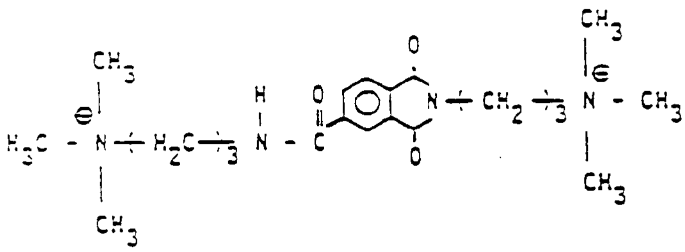 | 2x $BF_4^{\ominus}$ |
| 12.1b | s.o. | 2x B 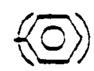 $_4^{\ominus}$ |
| 13.1a | 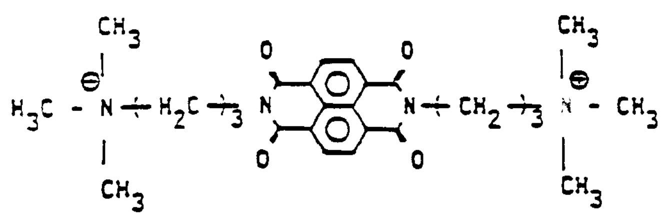 | 2x $BF_4^{\ominus}$ |
| 13.1b | s.o. | 2x $PF_6^{\ominus}$ |
| 13.1c | s.o. | 2x B 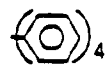 $_4^{\ominus}$ |
| 13.1d | s.o. | 2x $SCN^{\ominus}$ |

| Verbindung | Kation | Anion |
|---|---|---|
| 13.1e | 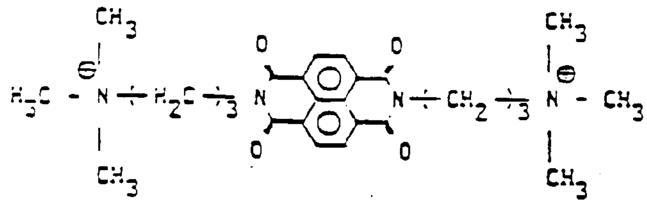 | 2x $CH_3SO_4^{\ominus}$ |
| 13.1f | s.o. | 2/3x $P[Mo_3O_{10}]_4^{3\ominus}$ |
| 13.2a | 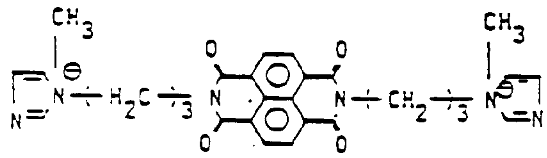 | 2x $BF_4^{\ominus}$ |
| 13.2b | s.o. | 2x B 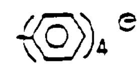 |
| 13.2c | s.o. | 2x $CH_3SO_4^{\ominus}$ |
| 13.3a | 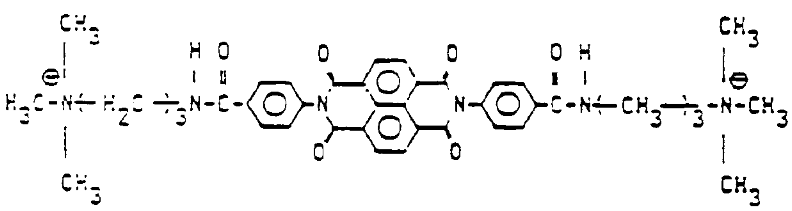 | 2x $BF_4^{\ominus}$ |
| 13.3b | s.o. | 2x B 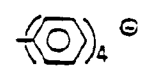 |
| 13.3.c | s.o. | 2x $CH_3SO_4^{\ominus}$ |
| 14.1a | 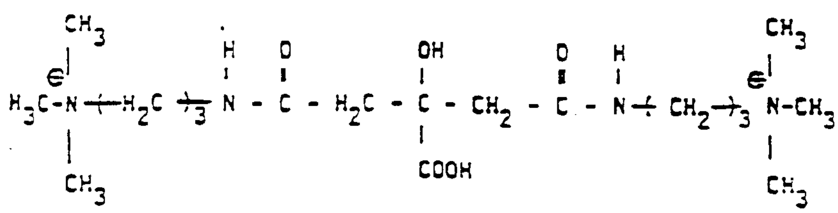 | 2x $BF_4^{\ominus}$ |
| 14.1b | s.o. | 2x B 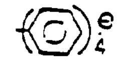 |

| Verbindung | Kation | Anion |
|---|---|---|
| 15.1a | 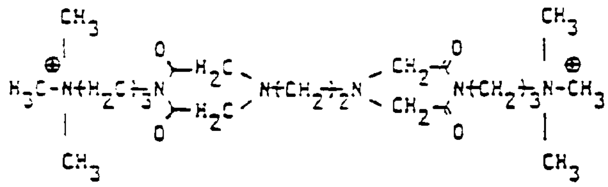 | 2 x $BF_4^{\ominus}$ |
| 15.1b | s. o. | 2 x 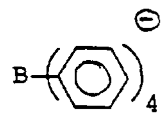 |
| 16.1a | 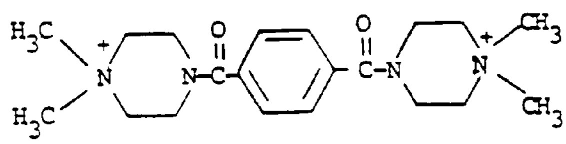 | 2 x $BF_4^-$ |
| 16.1b | s. o. | 2 · 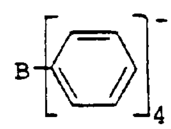 |

Die Herstellung der Verbindungen der allgemeinen Formeln (I) bis (III) geschieht in an sich bekannter Weise und ist in der Literatur ausführlich beschrieben (z.B. Houben-Weyl, "Methoden der Organischen Chemie", Georg Thieme Verlag, 1985, Band E 5, Teil 2, S. 924 - 1134 und a.a. Ort, 1958, Band 11/2, S. 591 - 630).

So erfolgt die Herstellung der Verbindungen (I) z.B. durch Umsetzung der aliphatischen, cycloaliphatischen, araliphatischen oder iso- bzw. heterocyclischen aromatischen Dicarbonsäuren oder geeigneter Dicarbonsäurederivate, wie z.B. deren Ester, Amide, Säurechloride oder Säureanhydride, mit Aminen oder Aminoverbindungen, die mindestens eine tertiäre und mindestens eine primäre oder sekundäre Aminogruppe enthalten, in einem inerten Reaktionsmedium oder in überschüssigem Amin als Reaktionsmedium und anschließender Bis-Protonierung mit einer anorganischen oder organischen Säure bzw. Bis-Quaternisierung mit einem geeigneten Quaternisierungsreagenz. Bevorzugt ist für die Herstellung der Verbindungen (I) der Einsatz von Dicarbonsäuredihalogeniden oder von Dicarbonsäurediestern als Ausgangsprodukte. Besonders bevorzugt ist als Herstellungsverfahren die Aminolyse von Dicarbonsäurediestern, insbesondere der Dimethyl- oder Diethylester, mit Aminen bei erhöhter Temperatur unter Abdestillieren des gebildeten Alkohols.

Die Herstellung der Verbindungen (II) erfolgt z.B. durch Umsetzung der aliphatischen, cycloaliphatischen, araliphatischen oder iso- bzw. heterocyclischen aromatischen Tetracarbonsäuren oder geeigneter Derivate, wie z.B. deren Ester, Amide, Säurechloride oder Säureanhydride, insbesondere deren Mono- oder Dianhydride, mit Aminen oder Aminoverbindungen, die mindestens eine tertiäre und mindestens eine primäre Aminogruppe enthalten, und anschließender Bis-Protonierung mit einer anorganischen oder organischen Säure bzw. Bis-Quaternisierung mit einem geeigneten Quaternisierungsreagenz. Die Umsetzung kann sowohl säurekatalysiert in wäßrigem Medium als auch in aliphatischen oder aromatischen Carbonsäuren oder in Gemischen aus Wasser und derartigen Carbonsäuren erfolgen. Eine besonders geeignete Carbonsäure ist die Essigsäure. Die Herstellung der Verbindungen (II) kann aber auch, gegebenenfalls säurekatalysiert, in, gegebenenfalls substituierten, aliphatischen oder aromatischen Kohlenwasserstoffen bei erhöhter Temperatur unter Auskreisen des

gebildeten Wassers erfolgen. Als besonders geeignete Kohlenwasserstoffe seien Toluol, Xylol, Chlorbenzol oder o-Dichlorbenzol genannt. Als Tetracarbonsäuren können prinzipiell alle diimidbildenden Tetracarbonsäuren verwendet werden. Bevorzugt sind aromatische Tetracarbonsäuren, bei denen jeweils zwei Carbonsäuregruppen in ortho- oder peri-Stellung zueinander stehen.

Die Herstellung der Verbindungen (III) erfolgt durch geeignete Kombination der füt die Verbidnungen (I) und (II) beschriebenen Verfahren.

Für die Bis-Protonierung bzw. Bis-Quaternisierung kommen prinzipiell alle geeigneten anorganischen und organischen Säuren bzw. alle geeigneten Alkylierungsmittel in Betracht. Besonders geeignet als Säuren sind Salzsäure, Schwefelsäure und Essigsäure. Als Alkylierungsmittel sind Alkylhalogenide und Dialkylsulfate bevorzugt, insbesondere Methyljodid, Methylchlorid bzw. Dimethyl- und Diethylsulfat. Als Reaktionsmedium für die Durchführung der Alkylierung kommen bevorzugt inerte Reaktionsmedien, wie beispielsweise Dimethylformamid oder aromatische Kohlenwasserstoffe in Betracht. Geeignet sind aber auch wasserfreie oder wasserhaltige Alkohole, wie beispielsweise Isobutanol oder das Isobutanol-Wasser-Azeotrop mit einem Wassergehalt von etwa 16%. In einzelnen Fällen kann die Quaternisierung auch in wäßrigem Medium durchgeführt werden.

Die Herstellung der verschiedenen Salze erfolgt durch Anionenaustausch, z.B. durch Ausfällen aus wäßrigem bzw. wäßrig-alkoholischem Medium, wie in den Herstellungsbeispielen beschrieben.

Der besondere Vorteil der erfindungsgemäß beanspruchten Verbindungen ist, daß sie farblos sind und einen hohen Ladungsteuereffekt aufweisen und daß dieser Ladungssteuereffekt über einen langen Aktivierzeitraum hinweg (bis zu 24 Std.) konstant ist. So zeigt z.B. ein Testtoner mit nur 1 Gewichts-% der Verbindung (1.1a) nach 10 min. eine Aufladung von +9 μC/g, nach 30 min. von +9 μC/g, nach 2 Std. von +6 μC/g und nach 24 Std. von +3 μC/g (Beispiel 3). Der hohe Ladungssteuereffekt wird umso deutlicher, wenn zum Vergleich z.B. das Aufladeverhalten des reinen Trägermaterials Dialec S309 betrachtet wird (Vergleichsbeispiel; 10 min: -4 μC/g; 30 min: -12 μC/g; 2 Std: -27 μC/g; 24 Std: -48 μC/g). Neben dem Einstellen der gewünschten Aufladungspolarität und -höhe müssen die erfindungsgemäß beanspruchten Verbindungen auch die hohe Ladungsdrift von über 40 μC/g konstant stellen.

Ein weiteren Vorteil der erfindungsgemäß beanspruchten Verbindungen ist, daß der Ladungssteuereffekt einer Verbindung lediglich durch Variation des Anions in kleinen Schritten verändert werden kann. Setzt man z.B. anstelle des in Beispiel 1 genannten $BF_4^{\ominus}$-Salzes das $PF_6^{\ominus}$-Salz desselben Kations ein (Verbindung 1.1b), so zeigt ein entsprechender Testtoner nach 10 min. eine Aufladung von +7 μC/g; nach 30 min. von +7 μC/g; nach 2 Std von +4 μC/g und nach 24 Std. von +3 μC/g (Beispiel 2). Darüberhinaus ist es auch möglich durch Wahl des geeigneten Anions das Vorzeichen des Ladungssteuereffekts umzupolen. Setzt man z.B. das

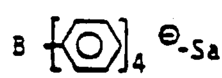 -Salz

des diskutierten Kations ein (Verbindung 1.1c), so zeigt sich anstelle des positiven ein negativer Ladungssteuereffekt (Beispiel 1; 10 min: -27 μC/g; 30 min: -27 μC/g; 2 Std: -25 μC/g; 24 Std: -23 μC/g). Darüberhinaus ist der Ladungssteuereffekt der erfindungsgemäß beanspruchten Verbindungen in hohem Maße unempfindlich gegen Luftfeuchtigkeitsschwankungen (Beispiel 3).

Für die Praxis von großer Bedeutung ist, daß die erfindungsgemäß beanspruchten Verbindungen chemisch inert und gut verträglich mit Trägermaterialien wie z.B. Styrol-Acrylaten, Polyestern, Epoxiden, Polyurethanen etc. sind. Zudem sind die Verbindungen thermostabil und können somit ohne Schwierigkeiten mit den gängigen Verfahren (Extrudieren, Kneten) unter den üblichen Bedingungen (Temperaturen zwischen 100°C und 200°C) in die gängigen Trägermaterialien eingearbeitet werden. Die Synthese der erfindungsgemß beanspruchten Verbindungen ist wenig aufwendig und die Produkte fallen in hoher Reinheit an.

Die erfindungsgemäß verwendeten Verbindungen werden in der Regel in einer Konzentration von etwa 0,01 bis etwa 30 Gewichtsprozent, vorzugsweise von etwa 0,1 bis etwa 5,0 Gewichtsprozent, in das jeweilige Trägermaterial in bekannter Weise, z.B. durch Einkneten oder Extrudieren, homogen eingearbeitet. Dabei können die Ladungssteuermittel für Toner bzw. ladungsverbessernde Mittel für Pulver und Lacke zur Oberflächenbeschichtung, insbesondere für triboelektisch bzw. elektrokinetisch versprühte Pulverlacke, als getrocknete und gemahlene Pulver, Dispersionen oder Lösungen, Preßkuchen, Masterbatch, als auf geeignete Träger wie z.B. Kieselgel, $TiO_2$, $Al_2O_3$ aus wäßriger oder nichtwäßriger Lösung aufgezogene Verbindungen oder in sonstiger Form zugegeben werden. Ebenso können die erfindungsgemäß eingesetzten Verbindungen grundsätzlich auch schon bei der Herstellung der jeweiligen Bindemittel zugegeben werden, d.h. im Verlauf von deren Polymerisation, Polyaddition oder Polykondensation.

Die Höhe der elektrostatischen Aufladung der elektrophotographischen Toner, in welche die erfindungsgemäß beanspruchten Ladungssteuermittel homogen eingearbeitet wurden, wurde an Standardtestsystemen unter gleichen Bedingungen (wie gleiche Dispergierzeiten, gleiche Teilchengrößenverteilung, gleiche Teilchenform) bei Raumtemperatur und 50% relativer Luftfeuchte gemessen. Für die Messung bei Raumtemperatur und 90% relativer Luftfeuchte wurde der jeweilige Toner 48 Stunden in einer Klimakammer konditioniert. Die Aktivierung des Toners in einem Zweikomponentenentwickler erfolgte durch Verwirbelung mit einem Carrier (3 Gewichtsteile Toner auf 97 Gewichtsteile Carrier) auf einer Rollbank (150 Umdrehungen pro Minute). Anschließend wurde an einem üblichen q/m-Meßstand die elektrostatische Aufladung gemessen (vgl. J.H. Dessauer, H.E. Clark, "Xerography and related Processes", Focal Press, N.Y., 1965, Seite 289). Bei der Bestimmung des q/m-Wertes ist die Teilchengröße von großem Einfluß, weshalb bei den durch Sichtung erhaltenen Tonerproben streng auf einheitliche Teilchengrößenverteilung geachtet wurde.

Die nachstehenden Beispiele dienen zur Erläuterung der Erfindung, ohne diese darauf zu beschränken. Die angegebenen Teile bedeuten Gewichtsteile.

HERSTELLUNGSBEISPIELE

Beispiel A

Amidbildung:

149,2 g (0,73 Mol) Terephthalsäuredichlorid werden in 3,5 l wasserfreiem Toluol verrührt und anschließend unter Kühlung 180,0 g (1,76 Mol) 3-Dimethylamino-1-propylamin bei 20 bis 30°C in 30 Minuten zugetropft. Man rührt 5 Stunden bei dieser Temperatur, erhitzt anschließend jeweils 1 bis 2 Stunden auf 50 bis 60°C bzw. 70 bis 80°C und erhitzt dann 4 Stunden zum Rückfluß. Das erhaltene Produkt wird bei 20 bis 30°C abgesaugt, mit Toluol gewaschen und bei 100°C im Vakuumschrank getrocknet. Man erhält 292,4 g (0,72 Mol) des Bisamides in Form des Bishydrochlorides. Das Produkt wird in 450 ml Wasser gelöst und bei 0 bis 5°C innerhalb von 30 Minuten mit 180 g 33%iger Natronlauge versetzt. Dabei fällt das Bisamid in grobkristalliner Form aus. Nach einstündigem Rühren bei 0 bis 5°C wird das Produkt abgesaugt, mit 90 ml Eiswasser gewaschen und bei 100°C im Vakuumschrank getrocknet.
Charakterisierung:
Weißes Pulver, Schmelzpunkt 172 - 174°C
$^1$H-NMR (in DMSO-$d_6$): 1,65 (Quintett, 4 Methylen-H), 2,13 (Singulett, 12 Methyl-H), 2,28 (Triplett, 4 Methylen-H), 3,30 (Quartett, 4 Methylen-H), 7,90 (Singulett, 4 Phenylen-H), 8,63 (Triplett, 2 Amid-H) ppm.

Quaternisierung

66,8 g (0,2 Mol) des Amids werden in 1,6 l Toluol verrührt und bei 20 bis 30°C innerhalb von 10 Minuten mit 100,8 g (0,8 Mol) Dimethylsulfat versetzt. Man rührt 1 Stunde bei 20 bis 30°C und erhitzt dann 5 Stunden am Rückfluß. Nach Abkühlung auf 20 bis 30°C wird das Produkt abgesaugt, mit Toluol gewaschen und bei 100°C im Vakuumschrank getrocknet.
Charakterisierung:
Weißes Pulver, Schmelzpunkt 180°C
$^1$H-NMR (in $D_2O$): 2,18 (Multiplett, 4 Methylen-H), 3,20 (Singulett, 18 Methyl-H), 3,50 (Multiplett, 8 Methylen-H), 3,75 (Singulett, 6 Mehthyl-H), 7,88 (Singulett, 4 Phenylen-H) ppm.

Anionenaustausch:

Zu 100 ml einer wäßrigen Lösung von 10,0 g (17 mMol) der quaternisierten Verbindung wird bei Raumtemperatur unter Rühren eine Lösung von 13,7 g (40 mMol) Natriumtetraphenylborat in 50 ml Wasser zugetropft. Dabei fällt Verbindung 1.1c als weißer Niederschlag aus. Der Niederschlag wird abgesaugt, mit Wasser gewaschen und im Umluftschrank bei 60°C getrocknet.
Charakterisierung:

Weißes Pulver, Schmelzpunkt 255°C
$^1$H-NMR (in DMSO-$d_6$): 1,98 (Multiplett, 4 Methylen-H), 3,03 (Singulett, 18 Methyl-H), 3,38 (Multiplett, 8 Methylen-H), 6,96 (Multiplett, 40 Phenyl-H), 7,95 (Singulett, 4 Phenylen-H), 8,64 (Triplett, 2 Amid-H) ppm.

Beispiel B

Amidbildung:

Die Amidbildung erfolgte wie in Beispiel A beschrieben.

Salzbildung

5,0 g (15 mMol) des Amids werden in 50 ml Wasser suspendiert und bis zum Erreichen von pH 7 mit 2 N Essigsäure versetzt, wobei das Amin in Lösung geht. Anschließend wird eine Lösung von 13,7 g (40 mMol) Natriumtetraphenylborat in 50 ml Wasser zugetropft, wobei das Produkt als dicker weißer Niederschlag ausfällt. Die Reaktionsmischung wird 30 min. bei Raumtemperatur gerührt, der Niederschlag abgesaugt, mit Wasser gewaschen, abschließend wird das Reaktionsprodukt, Verbindung 1.2c bei 60°C im Umluftschrank getrocknet.
Charakterisierung:
Weißes Pulver, Schmelzpunkt 197°C
$^1$H-NMR (in DMSO-$d_6$): 1,85 (Multiplett, 4 Methylen-H), 2,71 (Singulett, 12 Methyl-H), 3,00 (Multiplett, 4 Methylen-H), 3,35 (Multiplett, 4 Methylen-H), 6,96 (Multiplett, 40 Phenyl-H), 7,93 (Singulett, 4 Phenylen-H), 8,63 (Triplett, 2 Amid-H) ppm.

Beispiel C

Amidbildung:

109,5 g (0,75 Mol) Bernsteinsäuredimethylester werden in 459 g (4,5 Mol) 3-Dimethylamino-1-propylamin gelöst. Dann erhitzt man 10 Stunden unter Rückfluß. Da die Siedetemperatur durch die rasch einsetzende Methanolabspaltung erheblich absinkt, sorgt man durch gelegentliches Abdestillieren eines Methanol-Amin-Gemisches dafür, daß die Temperatur in der Gasphase oberhalb von 125°C bleibt. Gegen Ende der Reaktionsdauer liegt die Temperatur in der Gasphase oberhalb von 130°C. Im Verlauf der Reaktion werden etwa 200 g Methanol-Amin-Gemisch abdestilliert. Danach wird auf 20 bis 30°C abgekühlt und das auskristallisierte Reaktionsprodukt abgesaugt. Aus dem Filtrat läßt sich durch dreifache Verdünnung mit Benzin weiteres Produkt ausfällen. Das Produkt wird mit Benzin aminfrei gewaschen und bei 100°C im Vakuumschrank getrocknet.
Charakterisierung:
Weißes Pulver, Schmelzpunkt 126 - 128°C
$^1$H-NMR (in DMSO-$d_6$): 1,48 (Quintett, 4 Methylen-H), 2,08 (Singulett, 12 Methyl-H), 2,20 (Triplett, 4 Methylen-H), 2,25 (Singulett, 4 Methylen-H), 3,05 (Quartett, 4 Methylen-H), 7,78 (Triplett, 2 Amid-H) ppm.

Quaternisierung:

85,8 g (0,3 Mol) des Amids werden in 610 ml wasserfreies Dimethylformamid eingetragen. Bei Raumtemperatur entsteht rasch eine klare Lösung. Dann läßt man bei 30 bis 40°C in etwa 15 Minuten 189 g (1,5 Mol) Dimethylsulfat zutropfen. Nach kurzer Zeit entsteht ein dicker Kristallbrei, der bei Erwärmung auf 60°C in eine gut rührbare Suspension übergeht. Es wird 5 Stunden bei 60 bis 70°C nachgerührt und das Produkt nach Abkühlung auf 0 bis 5°C abgesaugt. Es wird gründlich mit Toluol gewaschen und im Vakuumschrank bei 100°C getrocknet.
Charakterisierung:
Weißes Pulver, Schmelzpunkt 152°C
$^1$H-NMR (in DMSO-$d_6$): 1,85 (Multiplett, 4 Methylen-H), 2,33 (Singulett, 4 Methyl-H), 3,05 (Singulett, 18 Methyl-H), 3,20 (Multiplett, 8 Methylen-H), 3,40 (Singulett, 6 Methyl-H), 7,95 (Triplett, 2 Amid-H) ppm.

Anionenaustausch:

10,0 g (18,5 mMol) des quaternisierten Produktes wurden, analog zur im Beispiel A beschriebenen Salzbildung, mit 13,7 g (40 mMol) Natriumtetraphenylborat ausgefällt, man erhält die Verbindung 6.1c.
Charakterisierung:
Weißes Pulver, Schmelzpunkt 245°C
$^1$H-NMR (in DMSO-$d_6$): 1,81 (Multiplett, 4 Methylen-H), 2,37 (Singulett, 4 Methylen-H), 3,02 (Singulett, 18 Methyl-H), 3,12 (Quartett, 4 Methylen-H), 3,24 (Multiplett, 4 Methylen-H), 6,97 (Multiplett, 40 Phenyl-H), 7,90 (Triplett, 2 Amid-H) ppm.

Beispiel D

Amidbildung:

Die Amidbildung erfolgte wie im Beispiel C beschrieben.

Salzbildung

Die Salzbildung mit 13,7 g (40 mMol) Natriumtetraphenylborat erfolgte analog zu der in Beispiel B beschriebenen Salzbildung, wobei 5,0 g (17,5 mMol) des in Beispiel C beschriebenen Amids eingesetzt wurden. Man erhält als Produkt Verbindung 6.2c.
Charakterisierung:
Weißes Pulver, Schmelzpunkt 183°C
$^1$H-NMR (in DMSO-$d_6$): 1,72 (Multiplett, 4 Methylen-H), 2,38 (Singulett, 4 Methylen-H), 2,68 (Singulett, 12 Methyl-H), 2,95 (Multiplett, 4 Methylen-H), 3,12 (Quartett, 4 Methylen-H), 7,01 (Multiplett, 40 Phenyl-H), 7,92 (Triplett, 2 Amid-H) ppm.

Beispiel E

Imidbildung:

218,0 g (1,0 Mol) Pyromellitsäuredianhydrid werden in 1,2 l Eisessig verrührt und bei 40 bis 50°C 306 g (3,0 Mol) 3-Dimethylamino-1-propylamin unter Kühlung zugetropft. Anschließend erhitzt man 3 Stunden zum Rückfluß, setzt 920 ml o-Dichlorbenzol zu und destilliert die Hauptmenge des Eisessigs ab. Dann erhitzt man unter Überleiten eines Stickstoffstromes und Abdestillieren des restlichen Eisessigs bis die Siedetemperatur von o-Dichlorbenzol erreicht ist und erhitzt 6 Stunden weiter am Rückfluß. Nach Abkühlung auf 20 bis 30°C wird das ausgefallene Produkt abgesaugt, mit Benzin gewaschen und bei 100°C im Vakuumschrank getrocknet.
Charakterisierung:

Weißes Pulver, Schmelzpunkt 186 - 188°C
$^1$H-NMR (in DMSO-$d_6$): 1,75 (Quintett, 4 Methylen-H), 2,08
(Singulett, 12 Methyl-H), 2,25
(Triplett, 4 Methylen-H), 3,68
(Triplett, 4 Methylen-H), 8,15
(Singulett, 2 Phenylen-H) ppm.

Quaternisierung:

77,2 g (0,2 Mol) des Imids werden in 600 ml Dimethylformamid verrührt und bei 30 bis 40°C 126,0 g (1,0 Mol) Dimethylsulfat unter Kühlung innerhalb von 20 Minuten zugetropft. Man erhitzt 5 Stunden auf 130 bis 135°C und saugt das Produkt nach Abkühlung auf 20 bis 30°C ab. Das Produkt wird mit Toluol gewaschen und bei 100°C im Vakuumschrank getrocknet.
Charakterisierung:
Weißes Pulver, Schmelzpunkt 197°C
$^1$H-NMR (in $D_2O$): 2,23 (Multiplett, 4 Methylen-H), 3,15
(Singulett, 18 Methyl-H), 3,48
(Multiplett, 4 Methylen-H), 3,73
(Singulett, 6 Methyl-H), 3,88
(Triplett, 4 Methylen-H), 8,33
(Singulett, 2 Phenylen-H) ppm.

Anionenaustausch:

Der Anionenaustausch mit 13,7 g (40 mMol) Natriumtetraphenylborat erfolgt analog zu dem in Beispiel A beschriebenen Anionenaustausch, wobei 10,0 g (16 mMol) der oben angeführten quaternisierten Verbindung eingesetzt wurden, man erhält als Produkt die Verbindung 11.1c.
Charakterisierung:
Weißes Pulver, Schmelzpunkt 295°C
$^1$H-NMR (in DMSO-$d_6$): 2,08 (Multiplett, 4 Methylen-H), 2,97
(Singulett, 18 Methyl-H), 3,36
(Multiplett, 4 Methylen-H), 3,70
(Triplett, 4 Methylen-H), 6,98
(Multiplett, 40 Phenyl-H), 8,28
(Singulett, 2 Phenylen-H) ppm.

Beispiel F

Imidbildung:

71,5 g (0,25 Mol) Naphthalin-1,4,5,8-tetracarbonsäure-1,8-monoanhydrid werden in 500 ml Eisessig verrührt und 93,8 g (0,75 Mol) N-(3-aminopropyl)-imidazol bei 40 bis 50°C unter leichter Kühlung zugetropft. Anschließend erhitzt man 6 Stunden am Rückfluß. Die entstandene Lösung des Reaktionsproduktes gießt man in 2,5 l Wasser und tropft dann bei 20 bis 30°C unter Kühlung 1,01 kg 33%ige Natronlauge zu, so daß das Produkt ausfällt. Es wird abgesaugt, gründlich mit Wasser gewaschen und bei 100°C im Vakuumschrank getrocknet.
Charakterisierung:
Weißes Pulver, Schmelzpunkt 260 - 263°C

Quaternisierung:

96,4 g (0,2 Mol) des Imids werden in 600 ml Dimethylformamid verrührt und 189,0 g (1,5 Mol) Dimethylsulfat werden innerhalb von 10 Minuten bei 30 bis 40°C unter leichter Kühlung zugetropft. Anschließend rührt man 5 Stunden bei 130 bis 135°C. Nach Abkühlung auf 20 bis 30°C wird das Produkt, Verbindung 13.2c abgesaugt, mit 100 ml Dimethylformamid, dann mit Toluol gewaschen und bei 100°C im Vakuumschrank getrocknet.
Charakterisierung:
Weißes Pulver, Schmelzpunkt 261°C

$^1H$-NMR (in $D_2O$): 2,35 (Quintett, 4 Methylen-H), 3,68 (Singulett, 6 Methyl-H), 3,95 (Singulett, 6 Methyl-H), 4,10 (Triplett, 4 Methylen-H), 4,39 (Triplett, 4 Methylen-H), 7,60 (Multiplett, 4 Imidazoyl-H), 8,40 (Singulett, 4 Naphthylen-H), 8,86 (Singulett, 2 Imidazoyl-H) ppm.

ANWENDUNGSBEISPIELE

Beispiel 1

Ein Teil des Terephthalsäurederivates 1.1c (Synthese der Verbindung siehe Herstellungsbeispiel A) wurde mittels eines Kneters der Fa. Werner & Pfleiderer (Stuttgart) 30 Minuten in 99 Teilen Tonerbindemittel (RDialec S 309 der Firma Diamond Shamrock (Styrol-Methacryl-Copolymer)) homogen eindispergiert. Anschließend wurde auf der Laboruniversalmühle 100 LU (Firma Alpine, Augsburg) gemahlen und dann auf dem Zentrifugalsichter 100 MZR (Firma Alpine) klassifiziert.

Die gewünschten Teilchenfraktion wurde mit einem Carrier aus mit Styrol-Methacryl-Copolymer 90:10 beschichteten Magnetit-Teilchen der Größe 50 bis 200μm des Typs "90μm Xerographic Carrier" der Firma Plasma Materials Inc. aktiviert.

Die Messung erfolgt an einem üblichen q/m-Meßstand (vgl. hierzu J.H. Dessauer, H.E. Clark "Xerography and related Processes", Focal Press, N.Y. 1965, Seite 289); durch Verwenden eines Siebes mit einer Maschenweite von 25μm (508 Mesh per inch), Fa. Gebrüder Kufferath, Düren, wurde sichergestellt, daß bei den Tonerausblasungen kein Carrier mitgerissen werden kann. Die Messungen erfolgten bei Raumtemperatur und 50% relativer Luftfeuchte, abweichende Versuchsbedingungen sind in den betreffenden Beispielen vermerkt.

In Abhängigkeit von der Aktivierdauer wurden folgende q/m-Werte [μC/g] gemessen:

| Aktivierdauer | [μC/g] |
|---|---|
| 10 min | - 27 |
| 30 min | - 27 |
| 2 Std | - 25 |
| 24 Std | - 23 |

Beispiel 2

1 Teil des Terephthalsäurederivates 1.1b (Synthese s.u.) wurde wie in Beispiel 1 beschrieben homogen in 99 Teile Tonerbindemittel eingearbeitet. In Abhängigkeit von der Aktivierdauer wurden folgende q/m-Werte [μC/g] gemessen:

| Aktivierdauer | [μC/g] |
|---|---|
| 10 min | + 7 |
| 30 min | + 7 |
| 2 Std | + 4 |
| 24 Std | + 3 |

Synthese

Die Herstellung des Amids und die Quaternisierung erfolgte analog Herstellungsbeispiel A.

Zum Anionenaustausch wurde anstelle von $NaB(Phenyl)_4$ das $KPF_6$-Salz eingesetzt.

Charakterisierung:

Weißes Pulver, Schmelzpunkt 265°C

$^1H$-NMR (in DMSO-$d_6$): 1,98 (Multiplett, 4 Methylen-H), 3,04 (Singulett, 18 Methyl-H), 3,37 (Multiplett, 8 Methylen-H), 7,92 (Singulett, 4 Phenylen-H), 8,63 (Triplett,

2 Amid-H) ppm.

Belspiel 3

1 Teil des Terephthalsäurederivates 1.1a (Synthese s.u.) wurde wie in Beispiel 1 beschrieben homogen in 99 Teile Tonerbindemittel eingearbeitet. In Abhängigkeit von der Aktivierdauer wurden folgende q/m-Werte [µC/g] gemessen:

| Aktivierdauer | [µC/g] |
|---|---|
| 10 min | + 9 |
| 30 min | + 9 |
| 2 Std | + 6 |
| 24 Std | + 3 |

Bei 90% relativer Luftfeuchte wurden folgende q/m-Werte [µ/C/g] bestimmt:

| Aktivierdauer | [µC/g] |
|---|---|
| 10 min | + 6 |
| 30 min | + 7 |
| 2 Std | + 7 |
| 24 Std | + 3 |

Synthese

Die Herstellung des Amids und die anschließende Quaternisierung erfolgte analog Herstellungsbeispiel A, wobei zum Erhalten des Niederschlages die Lösung auf 30 ml eingeengt und auf 2°C abgekühlt wird.

Zum Anionenaustausch wurde anstelle von NaB-(Phenyl)$_4$ ein $NaBF_4$-Salz eingesetzt.

Charakterisierung:

Weißes Pulver, Schmelzpunkt 228°C

$^1$H-NMR (in DMSO-$d_6$): 1,99 (Multiplett, 4 Methylen-H), 3,05 (Singulett, 18 Methyl-H), 3,38 (Multiplett, 8 Methylen-H), 7,93 (Singulett, 4 Phenylen-H), 8,62 (Triplett, 2 Amid-H) ppm.

Beispiel 4

1 Teil des Terephthalsäurederivates 1.2c (Synthese siehe Herstellungsbeispiel B) wurde wie in Beispiel 1 beschrieben homogen in 99 Teile Tonerbindemittel eingearbeitet. In Abhängigkeit von der Aktivierdauer wurden folgende q/m-Werte [µC/g] gemessen:

| Aktivierdauer | [µC/g] |
|---|---|
| 10 min | - 27 |
| 30 min | - 32 |
| 2 Std | - 32 |
| 24 Std | - 31 |

Beispiel 5

Ein Teil des Bernsteinsäurederivates 6.1c (Synthese siehe Herstellungsbeispiel C) wurde wie in Beispiel 1 beschrieben homogen in 99 Teile Tonerbindemittel eingearbeitet. In Abhängigkeit von der Aktivierdauer wurden folgende q/m-Werte [µC/g] gemessen:

| Aktivierdauer | µC/g |
|---|---|
| 10 min | - 31 |
| 30 min | - 34 |
| 2 Std | - 33 |
| 24 Std | - 22 |

Beispiel 6

Ein Teil des Bernsteinsäurederivates 6.1b (Synthese s.u.) wurde wie in Beispiel 1 beschrieben homogen in 99 Teile Tonerbindemittel eingearbeitet. In Abhängigkeit von der Aktivierdauer wurden folgende q/m-Werte [µC/g] gemessen:

| Aktivierdauer | [µC/g] |
|---|---|
| 10 min | - 5 |
| 30 min | - 7 |
| 2 Std | - 8 |
| 24 Std | - 7 |

Synthese

Die Herstellung des Amids und die Quaternisierung erfolgte analog Herstellungsbeispiel C.

Zum Anionenaustausch wurde anstelle von $NaB(Phenyl)_4$ ein $KPF_6$-Salz eingesetzt.

Charakterisierung:

Weißes Pulver, Schmelzpunkt 208°C

$^1$H-NMR (in DMSO-$d_6$): 1,83 (Multiplett, 4 Methylen-H), 2,35 (Singulett, 4 Methylen-H), 3,02 (Singulett, 18 Methyl-H), 3,12 (Quartett, 4 Methylen-H), 8,25 (Multiplett, 4 Methylen-H), 7,86 (Triplett, 2 Amid-H) ppm.

Beispiel 7

Ein Teil des Bernsteinsäurederivates 6.2c (Amidbildung siehe Herstellungsbeispiel C, Salzbildung analog Herstellungsbeispiel B) wurde wie in Beispiel 1 beschrieben homogen in 99 Teile Tonerbindemittel eingearbeitet.

In Abhängigkeit von der Aktivierdauer wurden folgende q/m-Werte [µC/g] gemessen:

| Aktivierdauer | [µC/g] |
|---|---|
| 10 min | - 35 |
| 30 min | - 38 |
| 2 Std | - 40 |
| 24 Std | - 32 |

Beispiel 8

Ein Teil des 1,4-Cyclohexandicarbonsäurederivates 3.2b (Synthese s.u.) wurde wie in Beispiel 1 beschrieben homogen in 99 Teile Tonerbindemittel eingearbeitet. In Abhängigkeit von der Aktivierdauer wurden folgende q/m-Werte [µC/g] gemessen:

| Aktivierdauer | [µC/g] |
|---|---|
| 10 min | - 6 |
| 30 min | - 8 |
| 2 Std | - 11 |
| 24 Std | - 12 |

Synthese

Die Herstellung des Amids und die Quaternisierung erfolgte analog Herstellungsbeispiel C, wobei anstelle von Bernsteinsäuredimethylester der 1,4-Cyclohexandicarbonsäuredimethylester eingesetzt wurde.

Zum Anionenaustausch wurde das $KPF_6$-Salz eingesetzt.

Charakterisierung:
Weißes Pulver, Schmelzpunkt 298°C
$^1$H-NMR (in DMSO-$d_6$): 1,35 (Multiplett, 4 Methylen-H), 1,8 (Multiplett, 8 Cyclohexylen-H), 2,05 (Multiplett, 2 Cyclohexylen-H), 3,05 (Singulett, 18 Methyl-H), 3,1 (Multiplett, 4 Methylen-H), 3,25 (Multiplett, 4 Methylen-H), 7,78 (Triplett, 2 Amid-H) ppm.

Beispiel 9

Ein Teil des 1,4-Cyclohexandicarbonsäurederivates 3.2c (Synthese s.u.) wurde wie in Beispiel 1 beschrieben homogen in 99 Teile Tonerbindemittel eingearbeitet. In Abhängigkeit von der Aktivierdauer wurden folgende q/m-Werte [µC/g] gemessen:

| Aktivierdauer | [µC/g] |
|---|---|
| 10 min | - 35 |
| 30 min | - 37 |
| 2 Std | - 37 |
| 24 Std | - 30 |

Synthese

Die Herstellung des Amids und die Quaternisierung erfolgte analog Herstellungsbeispiel C, wobei anstelle von Bernsteinsäuredimethylester der 1,4-Cyclohexandicarbonsäuredimethylester eingesetzt wurde.

Zum Anionenaustausch wurde anstelle von $KPF_6$ das NaB(Phenyl)$_4$-Salz eingesetzt.

Charakterisierung:
Weißes Pulver, Schmelzpunkt 255°C
$^1$H-NMR (in DMSO-$d_6$): 1,35 (Multiplett, 4 Methylen-H), 1,8 (Multiplett, 8 Cyclohexylen-H), 2,05 (Multiplett, 2 Cyclohexylen-H), 3,03 (Singulett, 18 Methyl-H), 3,1 (Multiplett, 4 Methylen-H), 3,25 (Multiplett, 4 Methylen-H), 6,95 (Multiplett, 40 Phenyl-H), 7,8 (Triplett, 2 Amid-H) ppm.

Beispiel 10

1 Teil des Pyromellitsäurederivates 11.1c (Synthese siehe Herstellungsbeispiel E) wurde wie in Beispiel 1 beschrieben homogen in 99 Teile Tonerbindemittel eingearbeitet.

In Abhängigkeit von der Aktivierdauer wurden folgende q/m-Werte [µC/g] gemessen:

| Aktivierdauer | [μC/g] |
|---|---|
| 10 min | - 13 |
| 30 min | - 20 |
| 2 Std | - 22 |
| 24 Std | - 18 |

Beispiel 11

1 Teil des Pyromellitsäurederivates 11.1a (Synthese s.u.) wurde wie in Beispiel 1 beschrieben homogen in 99 Teile Tonerbindemittel eingearbeitet. In Abhängigkeit von der Aktivierdauer wurden folgende q/m-Werte [μC/g] gemessen:

| Aktivierdauer | [μC/g] |
|---|---|
| 10 min | - 3 |
| 30 min | - 5 |
| 2 Std | - 8 |
| 24 Std | - 9 |

Synthese

Die Herstellung des Imids und die Quaternisierung erfolgte analog Herstellungsbeispiel E.

Zum Anionenaustausch wurde anstelle von NaB-(Phenyl)$_4$ ein $NaBF_4$-Salz eingesetzt.

Charakterisierung:

Weißes Pulver, Schmelzpunkt> 300°C

$^1$H-NMR (in DMSO-$d_6$): 2,12 (Multiplett, 4 Methylen-H), 3,05 (Singulett, 18 Methyl-H), 3,35 (Multiplett, 4 Methylen-H), 3,71 (Triplett, 4 Methylen-H), 8,26 (Singulett, 2 Phenylen-H) ppm.

Beispiel 12

1 Teil des Pyromellitsäurederivates 11.1d (Synthese s.u.) wurde wie in Beispiel 1 beschrieben homogen in 99 Teile Tonerbindemittel eingearbeitet. In Abhängigkeit von der Aktivierdauer wurden folgende q/m-Werte [μC/g] gemessen:

| Aktivierdauer | [μC/g] |
|---|---|
| 10 min | + 9 |
| 30 min | + 8 |
| 2 Std | + 4 |
| 24 Std | + 0,3 |

Synthese

Die Herstellung des Imids und die Quaternisierung erfolgte analog Herstellungsbeispiel E.

Zum Anionenaustausch wurde anstelle von NaB-(Phenyl)$_4$ ein KSCN-Salz eingesetzt.

Charakterisierung:

Weißes Pulver, Schmelzpunkt 285°C

$^1$H-NMR (in DMSO-$d_6$): 2,10 (Multiplett, 4 Methylen-H), 3,06 (Singulett, 18 Methyl-H) 3,41 (Multiplett, 4 Methylen-H), 3,70 (Triplett, 4 Methylen-H), 8,24 (Singulett, 2 Phenylen-H) ppm.

Beispiel 13

1 Teil des Pyromellitsäurederivates 11.1b (Synthese s.u.) wurde wie in Beispiel 1 beschrieben homogen in 99 Teile Tonerbindemittel eingearbeitet. In Abhängigkeit von der Aktivierdauer wurden folgende q/m-Werte [µC/g] gemessen:

| Aktivierdauer | [µC/g] |
|---|---|
| 10 min | - 3 |
| 30 min | - 8 |
| 2 Std | - 11 |
| 24 Std | - 11 |

Synthese

Die Herstellung des Imids und die Quaternisierung erfolgte analog Herstellungsbeispiel E.
Zum Anionenaustausch wurde anstelle von $NaB(Phenyl)_4$ das $KPF_6$-Salz eingesetzt.
Charakterisierung:
Weißes Pulver, Schmelzpunkt > 300°C
$^1$H-NMR (in DMSO-$d_6$): 2,08 (Multiplett, 4 Methylen-H), 3,03 (Singulett, 18 Methyl-H), 3,38 (Multiplett, 4 Methylen-H), 3,72 (Triplett, 4 Methylen-H), 8,27 (Singulett, 2 Phenylen-H) ppm.

Beispiel 14

1 Teil des Pyromellitsäurederivates 11.1a (Synthese s.u.) wurde wie in Beispiel 1 beschrieben homogen in 99 Teile Tonerbindemittel eingearbeitet. In Abhängigkeit von der Aktivierdauer wurden folgende q/m-Werte [µC/g] gemessen:

| Aktivierdauer | [µC/g] |
|---|---|
| 10 min | - 7 |
| 30 min | - 16 |
| 2 Std | - 25 |
| 24 Std | - 32 |

Synthese

Die Herstellung des Imids und die Quaternisierung erfolgte analog Herstellungsbeispiel E.
Zum Anionenaustausch wurde anstelle von $NaB(Phenyl)_4$ ein

$$Na_3[P(Mo_3O_{10})_4]\text{-Salz}$$

eingesetzt.
Charakterisierung:
Gelbliches Pulver, Schmelzpunkt > 300°C
$^1$H-NMR (in DMSO-$d_6$): 2,13 (Multiplett, 4 Methylen-H), 3,10 (Singulett, 18 Methyl-H), 3,42 (Multiplett, 4 Methylen-H), 3,73 (Triplett, 4 Methylen-H), 8,22 (Singulett, 2 Phenylen-H) ppm.

Beispiel 15

1 Teil des Pyromellitsäurederivates 11.1e (die Synthese des Methylsulfatsalzes ist in Herstellungsbeispiel E beschrieben) wurde wie in Beispiel 1 beschrieben homogen in 99 Teile Tonerbindemittel eingearbeitet. In

Abhängigkeit von der Aktivierdauer wurden folgende q/m-Werte [µC/g] gemessen:

| Aktivierdauer | [µC/g] |
|---|---|
| 10 min | + 7 |
| 30 min | + 7 |
| 2 Std | + 6 |
| 24 Std | + 3 |

Beispiel 16

1 Teil des Naphthalintetracarbonsäurederivates 13.2c (Synthese siehe Herstellungsbeispiel F) wurde wie in Beispiel 1 beschrieben homogen in 99 Teile Tonerbindemittel eingearbeitet. In Abhängigkeit von der Aktivierdauer wurden folgende q/m-Werte [µC/g] gemessen:

| Aktivierdauer | [µC/g] |
|---|---|
| 10 min | + 12 |
| 30 min | + 10 |
| 2 Std | + 7 |
| 24 Std | + 4 |

Beispiel 17

1 Teil des Naphthalintetracarbonsäurederivates 13.3c (Synthese s.u.) wurde wie in Beispiel 1 beschrieben homogen in 99 Teile Tonerbindemittel eingearbeitet.

In Abhängigkeit von der Aktivierdauer wurden folgende q/m-Werte [µC/g] gemessen:

| Aktivierdauer | [µC/g] |
|---|---|
| 10 min | - 1 |
| 30 min | - 1 |
| 2 Std | - 7 |
| 24 Std | - 10 |

Synthese

Die Herstellung des Imids und die Quaternisierung erfolgte analog Herstellungsbeispiel F wobei als Aminkomponente 2 Teile

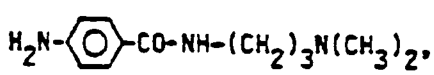

hergestellt durch Umsetzung von 4-Nitrobenzoylchlorid mit 3-Dimethylamino-1-propylamin und anschließender Reduktion der Nitrogruppe, eingesetzt wurden.

Charakterisierung:

Weißes Pulver, Schmelzpunkt>300°C (Zersetzung)

$^1$H-NMR (in DMSO-$d_6$): 2,6 (Multiplett, 4 Methylen-H), 3,5 (Singulett, 18 Methyl-H), 3,8 (Multiplett, 8 Methylen-H), 4,1 (Singulett, 6 Methyl-H), 7,6 (Multiplett, 4 Phenyl-H), 8,2 (Multiplett, 4 Phenyl-H), 8,9 (Singulett, 4 Naphthyl-H) ppm.

Beispiel 18

1 Teil des Naphthalintetracarbonsäurederivates 13.1a (Synthese s.u.) wurde wie in Beispiel 1 beschrieben homogen in 99 Teile Tonerbindemittel eingearbeitet.

In Abhängigkeit von der Aktivierdauer wurden folgende q/m-Werte [µC/g] gemessen:

| Aktivierdauer | [µC/g] |
|---|---|
| 10 min | + 7 |
| 30 min | + 4 |
| 2 Std | + 1 |
| 24 Std | - 2 |

Synthese

Die Herstellung des Imids und die Quaternisierung erfolgte wie in Herstellungsbeispiel F beschrieben, wobei als Aminkomponente 2 Teile 3-Dimethylamino-1-propylamin eingesetzt wurden. Der Anionenaustausch erfolgte analog wie in Beispiel A beschrieben mit $NaBF_4$.

Charakterisierung:

Weißes Pulver, Schmelzpunkt > 300°C

$^1$H-NMR (in DMSO-$d_6$): 2,20 (Multiplett, 4 Methylen-H), 3,05 (Singulett, 18 Methyl-H), 3,46 (Multiplett, 4 Methylen-H), 4,19 (Triplett, 4 Methylen-H), 8,68 (Singulett, 2 Naphthylen-H) ppm.

Beispiel 19

1 Teil des Naphthalintetracarbonsäurederivates 13.1c (Synthese s.u.) wurde wie in Beispiel 1 beschrieben homogen in 99 Teile Tonerbindemittel eingearbeitet.

In Abhängigkeit von der Aktivierdauer wurden folgende q/m-Werte [µC/g] gemessen:

| Aktivierdauer | [µC/g] |
|---|---|
| 10 min | - 3 |
| 30 min | - 7 |
| 2 Std | - 11 |
| 24 Std | - 14 |

Synthese

Die Herstellung des Imids und die Quaternisierung erfolgte wie in Herstellungsbeispiel F beschrieben, wobei als Aminkomponente 2 Teile 3-Dimethylamino-1-propylamin eingesetzt wurden.

Der Anionenaustausch erfolgte wie in Beispiel A beschrieben mit NaB(Phenyl)$_4$.

Charakterisierung:

Weißes Pulver, Schmelzpunkt > 287°C (Zersetzung)

$^1$H-NMR (in DMSO-$d_6$): 2,13 (Multiplett, 4 Methylen-H), 3,00 (Singulett, 18 Methyl-H), 3,45 (Multiplett, 4 Methylen-H), 4,16 (Triplett, 4 Methylen-H), 6,90 (Multiplett, 40 Phenyl-H), 8,71 (Singulett, 2 Naphthylen-H) ppm.

Vergleichsbeispiel zu den Beispielen 1 bis 19

100 Teile des in Beispiel 1 beschriebenen Tonerbindemittels Dialec S 309 wurden ohne weitere Zusatzstoffe wie in Beispiel 1 beschrieben 30 min in einem Kneter geknetet, und anschließend gemahlen, klassifiziert und an einem q/m-Meßstand vermessen.

In Abhängigkeit von der Aktivierdauer wurden folgenden q/m-Werte [µC/g) bestimmt:

| Aktivierdauer | [µC/g] |
|---|---|
| 10 min | - 4 |
| 30 min | - 12 |
| 2 Std | - 27 |
| 24 Std | - 48 |

Beispiel 20

1 Teil des in Beispiel 3 eingesetzten Terephthalsäurederivates 1.1a wurde analog wie in Beispiel 1 beschrieben homogen in 99 Teile eines Pulverlackbindemittels (®Alftalat AN 757 der Hoechst AG, Polyesterharz) eingearbeitet. In Abhängigkeit von der Aktivierdauer wurden folgende q/m-Werte [µC/g] gemessen:

| Aktivierdauer | [µC/g] |
|---|---|
| 10 min | - 7 |
| 30 min | - 8 |
| 2 Std | - 8 |
| 24 Std | - 6 |

Vergleichsbeispiel zu Beispiel 20

100 Teile des in Beispiel 20 beschriebenen Pulverlackbindemittels Alftalat AN 757 wurden ohne weitere Zusatzstoffe wie in Beispiel 1 beschrieben 30 min in einem Kneter geknetet, und anschließend gemahlen, klassifiziert und an einem q/m-Meßstand vermessen. In Abhängigkeit von der Aktivierdauer wurden folgende q/m-Werte [µC/g] vermessen

| Aktivierdauer | [µC/g] |
|---|---|
| 10 min | - 35 |
| 30 min | - 32 |
| 2 Std | - 24 |
| 24 Std | - 13 |

Beispiel 21

1 Teil der Verbindung 16.1a (Synthese s.u.) wurde, wie in Beispiel 1 beschrieben,homogen in 99 Teile Dialec S 309 eingearbeitet. In Abhängigkeit von der Aktivierdauer wurden folgende q/m-Werte [µC/g] gemessen:

| Aktivierdauer | [µC/g] |
|---|---|
| 10 min. | +21 |
| 30 min. | +19 |
| 2 Std. | +16 |
| 24 Std. | + 9 |

Synthese

101,5 g (0,5 Mol) Terephthalsäuredichlorid werden in 2,3 l Toluol gelöst und unter Kühlung bei 20 bis 30 °C 120 g (1,2 Mol) N-Methylpiperazin zugetropft. Man rührt 1 Stunde bei 20 bis 30 °C, heizt anschließend über 2 Stunden bis zum Rückfluß hoch und kocht 4 Stunden am Rückfluß. Nach Abkühlen auf Raumtemperatur

wird das Produkt abgesaugt, mit Toluol gewaschen und getrocknet. Das trockene Produkt wird in 400 ml Wasser gelöst, mit Aktivkohle und Kieselgur geklärt und das Bisamid durch Zugabe von 33%iger NaOH bei 0 bis 5 °C ausgefällt. Das Bisamid wird abgesaugt, mit Wasser gewaschen und bei 100 °C im Vakuum getrocknet. 66 g (0,2 Mol) des trockenen Bisamids werden in 640 ml Dimethylformamid gelöst und 76 ml (0,8 Mol) Dimethylsulfat werden bei Raumtemperatur unter leichter Kühlung über 15 Minuten zugetropft.

Anschließend wird 5 Stunden auf 60 bis 70 °C erwärmt und dann bei 0 bis 5 °C das Produkt abgesaugt, mit Toluol gewaschen und bei 100 °C im Vakuum getrocknet.

| | |
|---|---|
| Ausbeute: | 109 g (quantitative Ausbeute) weißes Pulver |
| Molekulargewicht: | 582 |
| Schmelzpunkt: | > 300°C |
| $^1$H-NMR (in $D_2O$): | 3,28 (Singulett, 12 Methyl-H), 3,53 (Multiplett, 8 H Piperazino-H), 3,73 (Singulett, Methyl-H des Methylsulfat-Anions, größtenteils zum Hydrogensulfat hydrolysiert), 3,88 (Multiplett, 4 Piperazino-H), 4,13 (Multiplett, 4 Piperazino-H), 7,60 (Singulett, 4 Phenylen-H) ppm. |

5,0 g (9 mMol) der obigen Verbindung werden bei Raumtemperatur in 20 ml Wasser gelöst. Anschließend werden 2,2 g (20 mMol) Natriumtetrafluoroborat in 25 ml Wasser langsam zugegeben, wobei die Reaktionsmischung durch ausfallende Kristalle sehr dick wird. Es wird auf 250 ml mit Wasser verdünnt und die farblosen Kristalle dann abgenutscht. Nach Waschen mit Wasser wird das Produkt bei 100 °C im Vakuumschrank getrocknet.

| | |
|---|---|
| Ausbeute: | 3,8 g (79,1 % der Theorie) der Verbindung 16.1a, farblose Kristalle |
| Molekulargewicht: | 534 |
| Schmelzpunkt: | > 300 °C |
| $^1$H-NMR (in DMSO-$d_6$): | 3,20 (Singulett, 12 Methyl-H), 3,48 (Singulett, 8 Piperazino-H), 3,83 (Singulett, 8 Piperazino-H), 7,56 (Singulett, 4 Phenylen-H) ppm. |

Beispiel 22

1 Teil der in Beispiel 21 beschriebenen Verbindung 16.1a wurde analog, wie in Beispiel 1 beschrieben, in 99 Teile Pulverlackbindemittel Alftalat AN 757 homogen eingearbeitet. In Abhängigkeit von der Aktivierdauer wurden folgende q/m-Werte [µC/g] gemessen:

| Aktivierdauer | [µC/g] |
|---|---|
| 10 min. | -26 |
| 30 min. | -22 |
| 2 Std. | -16 |
| 24 Std. | - 9 |

Beispiel 23

1 Teil der in Beispiel 21 beschriebenen Verbindung 16.1a wurde analog, wie in Beispiel 1 beschrieben, in 99 Teile Pulverlackbindemittel Crylcoat 430 (carboxylgruppenhaltiges Polyesterharz der Firma UCB, Belgien) homogen eingearbeitet. In Abhängigkeit von der Aktivierdauer wurden folgende q/m-Werte [µC/g] gemessen:

| Aktivierdauer | [µC/g] |
|---|---|
| 10 min. | -7 |
| 30 min. | -8 |
| 2 Std. | -9 |
| 24 Std. | -7 |

Vergleichsbeispiel zu Beispiel 23

100 Teile des in Beispiel 23 beschriebenen Pulverlackbindemittels Crylcoat 430 wurden ohne weitere Zusatzstoffe, wie in Beispiel 1 beschrieben, 30 min. in einem Kneter geknetet, und anschließend gemahlen, Klassifiziert und an einem q/m-Stand vermessen. In Abhängigkeit von der Aktivierdauer wurden folgende q/m-Werte [µC/g] gemessen:

| Aktivierdauer | [µC/g] |
|---|---|
| 10 min. | -20 |
| 30 min. | -15 |
| 2 Std. | - 9 |
| 24 Std. | - 7 |

Beispiel 24

Ein Teil der Verbindung 16.1b (Synthese s. u.) wurde, wie in Beispiel 1 beschrieben, homogen in 99 Teile Dialec S 309 eingearbeitet. In Abhängigkeit von der Aktivierdauer wurden folgende q/m-Werte [µC/g] gemessen:

| Aktivierdauer | [µC/g] |
|---|---|
| 10 min. | -19 |
| 30 min. | -22 |
| 2 Std. | -25 |
| 24 Std. | -21 |

Synthese

Es wird wie in Herstellungsbeispiel 21 verfahren, mit dem Unterschied, daß anstelle von Natriumtetrafluoroborat 7,0 g (20 mMol) Natriumetraphenylborat, gelöst in 20 ml Wasser, verwendet werden.

| | |
|---|---|
| Ausbeute: | 7,6 g (84,6 % der Theorie) der Verbindung 16.1b, weißes Pulver |
| Molekulargewicht: | 998 |
| Schmelzpunkt: | 292 °C (Zersetzung) |
| $^1$H-NMR (in DMSO-$d_6$): | 3,19 (Singulett, 12 Methyl-H), 3,46 (Singulett, 8 Piperazino-H), 3,82 (Singulett, 8 Piperazino-H), 7,03 (Multiplett, 40 Phenyl-H), 7,55 (Singulett, 4 Phenylen-H) ppm. |

## Patentansprüche

1. Verwendung von biskationischen Säureamid- und- imidderivaten der allgemeinen Formel (I) und/oder (II) und/oder (III)

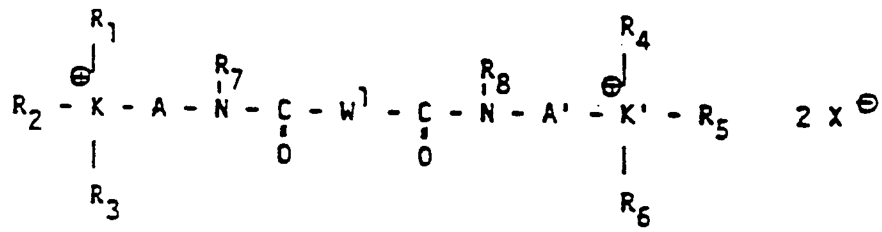

(I)

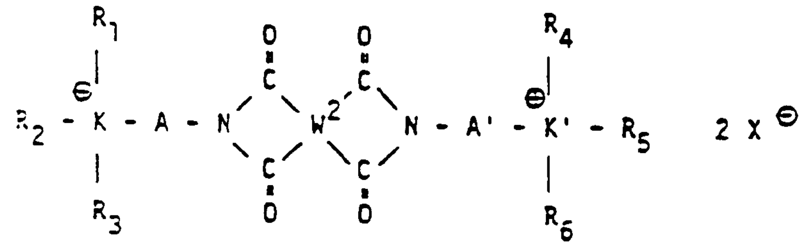

(II)

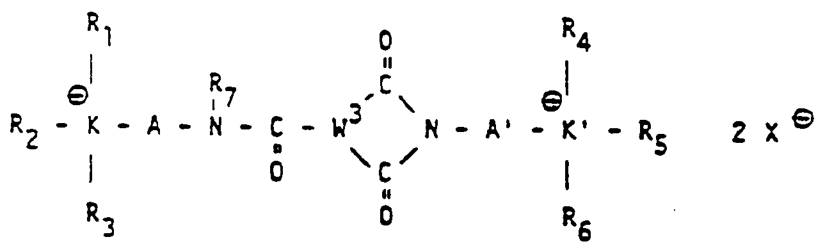

(III)

worin $R_1$ bis $R_8$ unabhängig voneinander jeweils ein Wasserstoffatom oder einen Kohlenwasserstoffrest, der durch Heteroatome unterbrochen sein kann, bedeuten, wobei die Reste $R_1$ und $R_2$ bzw. $R_4$ und $R_8$ sich unabhängig voneinander unter Einbeziehung von K bzw. K′ zu einem Ringsystem zusammen schließen können, und für den Fall, daß $R_1$ oder $R_2$ bzw. $R_4$ oder $R_5$ in diesem Zusammenhang eine Doppelbindung zu K oder K′ ausbilden, $R_3$ bzw. $R_6$ gegenstandslos sind, und/oder wobei einer der Reste $R_1$, $R_2$ oder $R_3$ sich mit $R_7$ bzw. einer der Reste $R_4$, $R_8$ oder $R_8$ sich mit $R_8$ zu einer aliphatischen Brücke aus 2 bis 5 C-Atomen zusammenschließen kann, und worin A und A′ sowie $W^1$, $W^2$ und $W^3$ unabhängig voneinander jeweils ein Brückenglied auf Basis eines kohlenwasserstoffes, der durch Heteroatome unterbrochen sein kann, sind, und wobei $W^1$ ein zweiwertiges, $W^2$ ein vierwertiges und $W^3$ ein dreiwertiges Brückenglied ist, und wobei $W^1$ auch eine direkte Bindung darstellen kann und K und K′ unabhängig voneinander jeweils ein Stickstoff-, Phosphor-, Arsen- oder Antimonatom darstellen kann und das Anion $X^{\ominus}$ das stöchiometrische Äquivalent eines oder mehrerer organischer oder anorganischer, gemischter oder nichtgemischter Anionen ist, wobei die Verbindung auch als Mischkristall mit verschiedenen Kationen der allgemeinen Formeln (I) bis (III) vorliegen kann, einzeln oder in Kombination als Ladungssteuermittel für Toner und Entwickler, die zum elektrophotographischen Kopieren bzw. Vervielfältigen von Vorlagen sowie zum Drucken von elektronisch, optisch oder magnetisch gespeicherten Informationen oder im Colorproofing eingesetzt werden sowie als Ladungssteuermittel für Pulver und Pulverlacke.

2. Verwendung von biskationischen Säureamid- und -imidderivaten gemäß Anspruch 1, dadurch gekennzeichnet, daß in den in Anspruch 1 genannten Formeln (I) bis (III) K und K′ jeweils ein Stickstoffatom darstellen.

3. Verwendung von biskationischen Säureamid- und -imidderivaten nach mindestens einem der Ansprüche 1 oder 2 dadurch gekennzeichnet, daß in den in Anspruch 1 genannten Formel (I) bis (III) $R_1$ bis $R_8$ unabhängig voneinander Wasserstoffatome, geradkettig oder verzweigte gesättigte oder ungesättigte Alkylgruppen von 1 bis 30 C-Atomen, Polyoxalkylengruppen der allgemeinen Formel -(Alkylen($C_1$-$C_5$)-O)$_n$R, worin R ein Wasserstoffatom oder eine Alkyl($C_1$-$C_4$)-Gruppe, eine Acylgruppe und n eine Zahl von 1 bis 10 ist, einen ein- oder mehrkernigen cycloaliphatischen Rest mit 5 bis 12 C-Atomen, einen ein- oder mehrkernigen aromatischen oder einen araliphatischen Rest darstellen, wobei solche aliphatischen, cycloaliphatischen, araliphatischen und aromatischen Reste durch Carbon- und/oder Sulfonsäuregruppen, bzw. deren Salze, Amide oder Ester-, Hydroxy-, Alkyl($C_1$-$C_4$)-, Alkoxy-($C_1$-$C_4$)- gruppen, primäre, sekundäre oder tertiäre Aminogruppen-, sowie durch Fluor-, Chlor- oder Bromatome, die aliphatischen Reste bevorzugt durch Fluoratome substituiert sein können, und wobei die genannten aliphatischen, cycloaliphatischen, araliphatischen oder aromatischen Ringsysteme ein oder mehrere Heteroatome enthalten können, und wobei $R_1$ und $R_3$ bzw. $R_4$ und $R_5$ sich unabhängig voneinander unter Einbeziehung von K bzw. K′ zu einem gesättigten oder ungesättigten, aromatischen oder nichtaromatischen 5- bis 7-gliedrigem Ringsystem zusammenschließen können, das weitere Heteroatome enthalten kann sowie substituiert und/oder durch Ankondensation von oder Verbrückung zu weiteren Ringsystemen modifiziert sein kann, und wobei für den Fall, daß $R_1$ oder $R_2$ bzw. $R_4$ oder $R_5$ eine Doppelbindung zu K bzw. K′ ausbilden, $R_3$ bzw. $R_5$ gegenstandlos sind.

4. Verwendung der biskationischen Säureamid- und -imidderivate nach mindestens einem der Ansprüche 1 bis 3 dadurch gekennzeichnet, daß in den in Anspruch 1 genannten Formeln (I) bis (III) A und A′ unabhängig voneinander jeweils ein geradkettiges oder verzweigtes, gesättigtes oder ungesättigtes, aliphatisches Brückenglied mit 1 bis 30 Kohlenstoffatomen, ein ein- oder mehrkerniges cycloaliphatisches Brückenglied, ein ein- oder mehrkerniges aromatisches Brückenglied oder araliphatisches Brückenglied, darstellen, wobei die aliphatischen, cycloaliphatischen, araliphatischen und aromatischen Brückenglieder durch Hydroxy-, Carbon- und/oder Sulfonsäuregruppen, bzw. deren Salze, Amide oder Ester, Alkyl($C_1$-$C_4$)-, Alkoxy($C_1$-$C_4$)-gruppen, primäre, sekundäre oder tertiäre Aminogruppen, sowie durch Fluor-, Chlor- oder Bromatome, die aliphatischen Brückenglieder bevorzugt durch Fluoratome, substituiert sein können, und wobei die genannten aliphatischen, cycloaliphatischen, aromatischen und araliphatischen Ringsysteme ein oder mehrere Stickstoff- und/oder Sauerstoff- und/oder Schwefelatome enthalten können.

5. Verwendung von biskationischen Säureamid- und -imidderivaten nach mindestens einem der Ansprüche 1 bis 4 dadurch gekennzeichnet, daß $W^1$, in den in Anspruch 1 genannten Formeln (I) bis (III) als zweiwertiges Brückenglied ein geradkettiges oder verzweigtes, gesättigtes oder ungesättigtes aliphatisches Brückenglied mit 1 bis 30 C-Atomen, ein Polyoxalkylenglied der allgemeinen Formel -$CH_2$-O-(Alkylen($C_1$-$C_5$)-O)$_m$$CH_2$-, wobei m eine Zahl von 0 bis 10 ist, ein ein- oder mehrkerniges cycloaliphatisches Brückenglied mit 5 bis 12 C-Atomen, ein ein- oder mehrkerniges aromatisches Brückenglied, oder ein araliphatisches Brückenglied darstellt, wobei die aliphatischen, cycloaliphatischen, araliphatischen und aromatischen Brückenglieder durch Carbon-, und/oder Sulfonsäuregruppen, bzw. deren Salze, Amide oder Ester, Hydroxy-, Alkyl($C_1$-$C_4$)-, Alkoxy($C_1$-$C_4$)-gruppen, primäre, sekundäre oder tertiäre Aminogruppen, sowie durch Fluor-, Chlor- oder Bromatome, die aliphatischen Brückenglieder bevorzugt durch Fluoratome substituiert sein können und wobei die aliphatischen Zwischenglieder sowie die cycloaliphatischen, die araliphatischen und die aromatischen Ringsysteme ein oder mehrere Stickstoff- und/oder Sauerstoff- und/oder Schwefelatome, enthalten können, und wobei $W^1$ auch eine direkte Bindung bedeuten kann, $W^2$ als vierwertiges Brückenglied ein geradkettiges oder verzweigtes, gesättigtes oder ungesättigtes aliphatisches Brückenglied mit 2 bis 30 C-Atomen, ein ein- oder mehrkerniges aromatisches, cycloaliphatisches Brückenglied mit 5 bis 12 C-Atomen, oder ein araliphatisches Brückenglied darstellt, wobei die aliphatischen, cycloaliphatischen, araliphatischen und aromatischen Brückenglieder durch Carbon- und/oder Sulfonsäuregruppen, bzw. deren Salze, Amide oder Ester, Hydroxy-, Alkyl($C_1$-$C_4$)-, Alkoxy($C_1$-$C_4$)-gruppen, primäre, sekundäre oder tertiäre Aminogruppen, sowie durch Fluor-, Chlor- oder Bromatome, die aliphatischen Brückenglieder bevorzugt durch Fluoratome substituiert sein können, und wobei die aliphatischen Brückenglieder sowie die cycloaliphatischen, die araliphatischen und die aromatischen Ringsysteme ein oder mehrere Stickstoff- und/oder Sauerstoff- und/oder Schwefelatome enthalten können,

und $W^3$ als dreiwertiges Brückenglied ein geradkettiges oder verzweigtes, gesättigtes oder ungesättigtes aliphatisches Brückenglied mit 2 bis 30 C-Atomen, ein ein- oder mehrkerniges cycloaliphatisches Brückenglied mit 5 bis 12 C-Atomen, ein ein- oder mehrkerniges aromatischen Brückenglied, oder ein araliphatischen Brückenglied darstellt, wobei die aliphatischen, araliphatischen und aromatischen

Brückenglieder durch Carbon- und/oder Sulfonsäuregruppen, bzw. deren Salze, Amide oder Ester, Hydroxy-, Alkyl($C_1$-$C_4$)-, Alkoxy($C_1$-$C_4$)-gruppen, primäre, sekundäre oder tertiäre Amingruppen, sowie durch Fluor-, Chlor- oder Bromatome, die aliphatischen Brückenglieder bevorzugt durch Fluoratome substituiert sein können und wobei die aliphatischen Brückenglieder sowie die cycloaliphatischen, die araliphatischen und die aromatischen Ringsysteme ein oder mehrere Stickstoff- und/oder Sauerstoff- und/oder Schwefelatome, enthalten können.

**6.** Verwendung von biskationischen Säureamid- und- imidderivaten nach mindestens einem der Ansprüche 1 bis 5 dadurch gekennzeichnet, in den in Anspruch 1 genannten Formeln (I) bis (III), daß das Anion $X^{\ominus}$ ein Halogenid, $PF_6^{\ominus}$, $SO_4^{2-}$, $HSO_4^{\ominus}$, Phosphat, $NO_3^{\ominus}$, Cyanat, Thiocyanat, $BF_4^{\ominus}$, $B(Aryl)_4^{\ominus}$, Phenolat, Nitrophenolat, Zinktetracyanat, Zinktetrathiocyanat, $CH_3OSO_3^{\ominus}$, $C_2H_5OSO_3^{\ominus}$ gesättigtes oder ungesättigtes, aliphatisches oder aromatisches Carboxylat oder Sulfonat, oder ein Wolframat-, Molybdat- sowie Heteropolysäureanion darstellt, wobei diese Anionen auch in gemischter Form vorliegen können.

**7.** Verwendung von biskationischen Säureamid- und -imidderivaten nach mindestens einem der Ansprüche 1 bis 6 dadurch gekennzeichnet, daß in den in Anspruch 1 genannten Formeln (I) bis (III) K und K′ jeweils ein Stickstoffatom bedeutet, $R_1$ bis $R_6$ entweder unabhängig voneinander jeweils ein Wasserstoffatom oder eine ($C_1$-$C_4$)-Alkylgruppe darstellen oder $R_1$ und $R_2$ bzw. $R_4$ und $R_5$ sich unabhängig voneinander unter Einbeziehung von K bzw. K′ zu einem gesättigten oder ungesättigten 5- oder 6-gliedrige heterocyclischen Ringsystem mit einem oder zwei Stickstoffatomen als Heterobestandteil zusammenschließen können, wobei dann $R_3$ bzw. $R_6$ unabhängig voneinander jeweils ein Wasserstoffatom oder eine ($C_1$-$C_4$)-Alkylgruppe darstellen, bzw. für den Fall, daß $R_1$ oder $R_2$ bzw. $R_4$ oder $R_5$ in diesem Zusammenhang eine Doppelbindung zu K oder K′ ausbilden, $R_3$ bzw. $R_5$ gegenstandslos sind, und $R_7$ und $R_5$ ein Wasserstoffatom bedeuten, und A und A′ unabhängig voneinander jeweils ein $(CH_2)_p$ Brückenglied mit p= 1 bis 4, ein Phenylen-, Naphthylen- oder

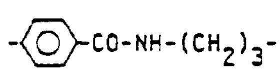

Brückenglied bedeutet, $W^1$ ein Phenylen-, Naphthylen, Cyclohexylen-, ein $(CH_2)_q$ Brückenglied mit q= 1 bis 12 oder ein $(CH_2$-$O(CH_2$-$CH_2$-$O)_r$-$CH_2$- Brückenglied mit r= 1 - 4, $W^2$ ein Phenylen- oder Naphthylen-Brückenglied, wobei die zur imidbildung benötigten Carboxylgruppen im Falle von Phenylen jeweils in ortho-Stellung, im Falle von Naphthylen in ortho- und/oder peri-Stellung zueinander stehen oder ein Ethylendiamintetramethylen-Brückenglied und $W^3$ ein Phenylen- oder Naphthylenbrückenenglied ist, und das Anion $X^{\ominus}$ ein $B(Aryl)_4^{\ominus}$, $BF_4^{\ominus}$, $PF_6^{\ominus}$, $SCN^{\ominus}$ oder $CH_3SO_4^{\ominus}$ bedeutet.

**8.** Verwendung von biskationischen Säureamid- und -imidderivaten nach mindestens einem der Ansprüche 1 bis 7, dadurch gekennzeichnet, daß man diese Verbindungen einzeln oder in Kombination in einer Konzentration von etwa 0,01 bis etwa 30 Gewichtsprozent einsetzt.

**9.** Verwendung von mindestens einer der in den Ansprüchen 1 bis 7 genannten Verbindungen als Bestandteil von Beschichtungen von Carriern, die in Entwicklern zum elektrophotographischen Kopieren oder Vervielfältigen von Vorlagen sowie zum Drucken von elektronisch, optisch oder magnetisch gespeicherten Informationen oder im Colorproofing eingesetzt werden.

**10.** Verwendung von in mindestens einer der Ansprüche 1 bis 7 genannten Verbindungen, einzeln oder in Kombination, als ladungsverbesserndes Mittel in Pulvern und Lacken zur Oberflächenbeschichtung von Gegenständen aus Metall, Holz, Kunststoff, Glas, Keramik, Beton, Textilmaterial, Papier oder Kautschuk, insbesondere in triboelektisch bzw. elektrokinetisch versprühten Pulverlacken.

## Claims

**1.** Use of biscationic acid amide and acid imide derivatives of the formula (I) and/or (II) and/or (III)

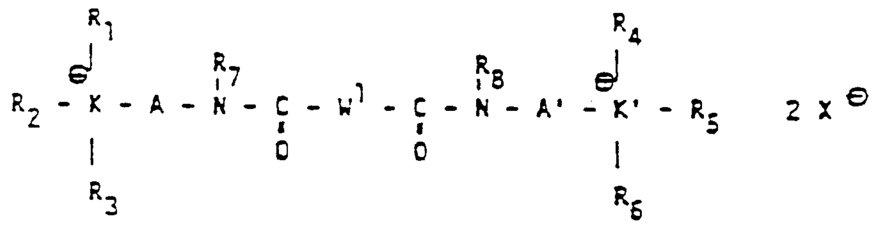

(I)

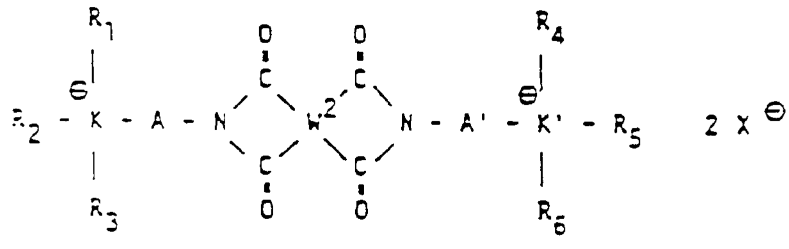

(II)

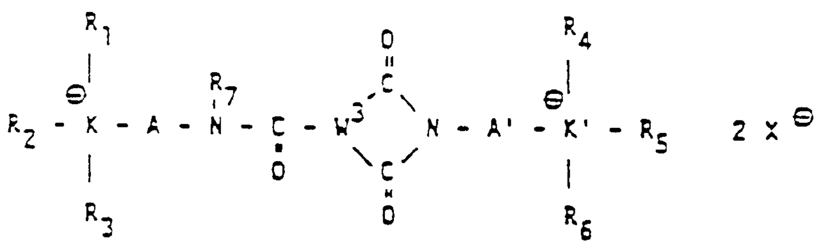

(III)

in which $R_1$ to $R_8$ independently of one another are each a hydrogen atom or a hydrocarbon radical, which can be interrupted by hetero atoms, and in which the radicals $R_1$ and $R_2$, or $R_4$ and $R_8$, independently of one another can be closed together, incorporating K or K′, to form a ring system, and in the case where $R_1$ or $R_2$, or $R_4$ or $R_5$, in this connection form a double bond to K or K′, $R_3$ and $R_8$ have no meaning, and/or in which one of the radicals $R_1$, $R_2$ or $R_3$ can be closed together with $R_7$, or one of the radicals $R_4$, $R_8$ or $R_6$ can be closed together with $R_8$, to form an aliphatic bridge of 2 to 5 carbon atoms, and in which A and A′ and $W^1$, $W^2$ and $W^3$ independently of one another are each a bridge member based on a hydrocarbon, which can be interrupted by hetero atoms, and in which $W^1$ is a divalent, $W^2$ a tetravalent and $W^3$ a trivalent bridge member, and in which $W^1$ can also be a direct bond and K and K′ independently of one another can each be a nitrogen, phosphorus, arsenic or antimony atom, and the anion $X^{\ominus}$ is the stoichiometric equivalent of one or more organic or inorganic, mixed or non-mixed anions, it also being possible for the compound to be present as a mixed crystal with various cations of the formulae (I) to (III), individually or in combination as charge controllers for toners and developers which are used for electrophotographic copying or multicopying of originals and for printing electronically, optically or magnetically stored data or in color proofing, and as charge controllers for powders and powder coatings.

2. Use of biscationic acid amide and acid imide derivatives as claimed in claim 1, wherein, in the formulae (I) to (III) mentioned in claim 1, K and K′ are each a nitrogen atom.

3. Use of biscationic acid amide and acid imide derivatives as claimed in at least one of claims 1 or 2, wherein, in the formulae (I) to (III) mentioned in claim 1, $R_1$ to $R_8$ independently of one another are hydrogen atoms, straight-chain or branched saturated or unsaturated alkyl groups having 1 to 30 carbon atoms, polyoxyalkylene groups of the formula $-(alkylene(C_1-C_5)-O)_nR$, in which R is a hydrogen atom or an alkyl($C_1$-$C_4$)

group or an acyl group and n is a number from 1 to 10, a mono- or polynuclear cycloaliphatic radical having 5 to 12 carbon atoms or a mono- or polynuclear aromatic or an araliphatic radical, and in which such aliphatic, cycloaliphatic, araliphatic and aromatic radicals can be substituted by carboxylic and/or sulfonic acid groups, or salts, amides or esters thereof, hydroxyl, alkyl($C_1$-$C_4$) or alkoxy ($C_1$-$C_4$) groups or primary, secondary or tertiary amino groups, and by fluorine, chlorine or bromine atoms, and the aliphatic radicals preferably by fluorine atoms, and in which the aliphatic, cycloaliphatic, araliphatic or aromatic ring systems mentioned can contain one or more hetero atoms, and in which $R_1$ and $R_3$, or $R_4$ and $R_5$, independently of one another can be closed together, incorporating K or K′, to form a saturated or unsaturated, aromatic or non-aromatic 5- to 7-membered ring system, which can contain further hetero atoms and can be substituted and/or modified by condensation of or bridging to further ring systems, and in which, in the case where $R_1$ or $R_2$, or $R_4$ or $R_5$, form a double bond to K or K′, $R_3$ and $R_6$ have no meaning.

4. Use of the biscationic acid amide and acid imide derivatives as claimed in at least one of claims 1 to 3, wherein, in the formulae (I) to (III) mentioned in claim 1, A and A′ independently of one another are each a straight-chain or branched, saturated or unsaturated, aliphatic bridge member having 1 to 30 carbon atoms, a mono- or polynuclear cycloaliphatic bridge member or a mono- or polynuclear aromatic bridge member or araliphatic bridge member, in which the aliphatic, cycloaliphatic, araliphatic and aromatic bridge members can be substituted by hydroxyl, carboxylic and/or sulfonic acid groups, or salts, amides or esters thereof, alkyl($C_1$-$C_4$) or alkoxy($C_1$-$C_4$) groups or primary, secondary or tertiary amino groups, and by fluorine, chlorine or bromine atoms, and the aliphatic bridge members preferably by fluorine atoms, and in which the aliphatic, cycloaliphatic, aromatic and araliphatic ring systems mentioned can contain one or more nitrogen and/or oxygen and/or sulfur atoms.

5. Use of biscationic acid amide and acid imide derivatives as claimed in at least one of claims 1 to 4, wherein $W^1$, in the formulae (I) to (III) mentioned in claim 1, as a divalent bridge member is a straight-chain or branched, saturated or unsaturated aliphatic bridge member having 1 to 30 carbon atoms, a polyoxyalkylene member of the formula $-CH_2-O-(alkylene(C_1-C_5)-O)_mCH_2-$, in which m is a number from 0 to 10, a mono- or polynuclear cycloaliphatic bridge member having 5 to 12 carbon atoms, a mono- or polynuclear aromatic bridge member or an araliphatic bridge member in which the aliphatic, cycloaliphatic, araliphatic and aromatic bridge members can be substituted by carboxylic and/or sulfonic acid groups, or salts, amides or esters thereof, hydroxyl, alkyl($C_1$-$C_4$) or alkoxy($C_1$-$C_4$) groups or primary, secondary or tertiary amino groups, and by fluorine, chlorine or bromine atoms, the aliphatic bridge members preferably by fluorine atoms, and in which the aliphatic intermediate members and the cycloaliphatic, the araliphatic and the aromatic ring systems can contain one or more nitrogen and/or oxygen and/or sulfur atoms, and in which $W^1$ can also be a direct bond, $W^2$ as a tetravalent bridge member is a straight-chain or branched, saturated or unsaturated aliphatic bridge member having 2 to 30 carbon atoms, a mono- or polynuclear aromatic or cycloaliphatic bridge member having 5 to 12 carbon atoms or an araliphatic bridge member, in which the aliphatic, cycloaliphatic, araliphatic and aromatic bridge members can be substituted by carboxylic and/or sulfonic acid groups, or salts, amides or esters thereof, hydroxyl, alkyl($C_1$-$C_4$) or alkoxy($C_1$-$C_4$) groups or primary, secondary or tertiary amino groups, and by fluorine, chlorine or bromine atoms, and the aliphatic bridge members preferably by fluorine atoms, and in which the aliphatic bridge members and the cycloaliphatic, the araliphatic and the aromatic ring systems can contain one or more nitrogen and/or oxygen and/or sulfur atoms, and $W^3$ as a trivalent bridge member is a straightchain or branched, saturated or unsaturated aliphatic bridge member having 2 to 30 carbon atoms, a mono- or polynuclear cycloaliphatic bridge member having 5 to 12 carbon atoms, a mono- or polynuclear aromatic bridge member or an araliphatic bridge member, in which the aliphatic, araliphatic and aromatic bridge members can be substituted by carboxylic and/or sulfonic acid groups, or salts, amides or esters thereof, hydroxyl, alkyl($C_1$-$C_4$) or alkoxy($C_1$-$C_4$) groups or primary, secondary or tertiary amine groups, and by fluorine, chlorine or bromine atoms, and the aliphatic bridge members preferably by fluorine atoms, and in which the aliphatic bridge members and the cycloaliphatic, the araliphatic and the aromatic ring systems can contain one or more nitrogen and/or oxygen and/or sulfur atoms.

6. Use of biscationic acid amide and acid imide derivatives as claimed in at least one of claims 1 to 5, wherein, in the formulae (I) to (III) mentioned in claim 1, the anion $X^{\ominus}$ is a halide, $pF_6^{\ominus}$, $SO_4^{2-}$, $HSO_4^{\ominus}$, phosphate, $NO_3^{\ominus}$, cyanate, thiocyanate, $BF_4^{\ominus}$, $B(aryl)_4^{\ominus}$, phenolate, nitrophenolate, zinc tetracyanate, zinc tetrathiocyanate, $CH_3OSO_3^{\ominus}$, $C_2H_5OSO_3^{\ominus}$ or saturated or unsaturated, aliphatic or aromatic carboxylate or sulfonate, or a tungstate, molybdate or heteropolyacid anion, it also being possible for these anions to be present in mixed form.

7. Use of biscationic acid amide and acid imide derivatives as claimed in at least one of claims 1 to 6, wherein, in the formulae (I) to (III) mentioned in claim 1, K and K′ are each a nitrogen atom, $R_1$ to $R_6$ either independently of one another are each a hydrogen atom or a $(C_1$-$C_4)$-alkyl group, or $R_1$ and $R_2$, or $R_4$ and $R_5$, independently of one another can be closed together, incorporating K or K′, to form a saturated or unsaturated 5- or 6-membered heterocyclic ring system having one or two nitrogen atoms as the hetero constituent, in which case $R_3$ and $R_5$ independently of one another are each a hydrogen atom or a $(C_1$-$C_4)$-alkyl group, or in the case where $R_1$ or $R_2$, or $R_4$ or $R_5$, in this connection form a double bond to K or K′, $R_3$ and $R_5$ have no meaning, and $R_7$ and $R_8$ are a hydrogen atom, and A and A′ independently of one another is each a $(CH_2)_p$ bridge member, where p is 1 to 4, or a phenylene, naphthylene or

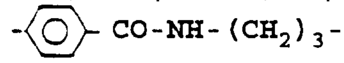

bridge member, $W^1$ is a phenylene, naphthylene, cyclo-hexylene or a $(CH_2)_q$ bridge member, where q is 1 to 12, or a $(CH_2$-$O(CH_2$-$CH_2$-$O)_r$-$CH_2$- bridge member, where r is 1 - 4, $W^2$ is a phenylene or naphthylene bridge member, in which the carboxyl groups required for imide formation are in each case in the ortho-position relative to one another in the case of phenylene and in the ortho- and/or peri-position relative to one another in the case of naphthylene, or an ethylenediaminetetramethylene bridge member, and $W^3$ is a phenylene or naphthylene bridge member, and the anion $X^{\ominus}$ is a $B(aryl)_4^{\ominus}$, $BF_4^{\ominus}$, $PF_6^{\ominus}$, $SCN^{\ominus}$ or $CH_3SO_4^{\ominus}$.

8. Use of biscationic acid amide and acid imide derivatives as claimed in at least one of claims 1 to 7, wherein these compounds are employed individually or in combination in a concentration of about 0.01 to about 30 percent by weight.

9. Use of at least one of the compounds mentioned in claims 1 to 7 as a constituent of coatings of carriers which are employed in developers for electrophotographic copying or multicopying of originals and for printing electronically, optically or magnetically stored data or in color proofing.

10. Use of compounds mentioned in at least one of claims 1 to 7, individually or in combination, as charge-improving agent in powders and paints for surface coating of objects of metal, wood, plastic, glass, ceramic, concrete, textile material, paper or rubber, in particular in triboelectrically or electrokinetically sprayed powder coatings.

## Revendications

1. Utilisation de dérivés d'amides et d'imides dicationiques de formule générale (I) et/ou (II) et/ou (III)

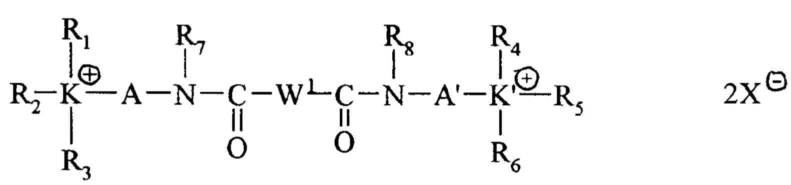

(I)

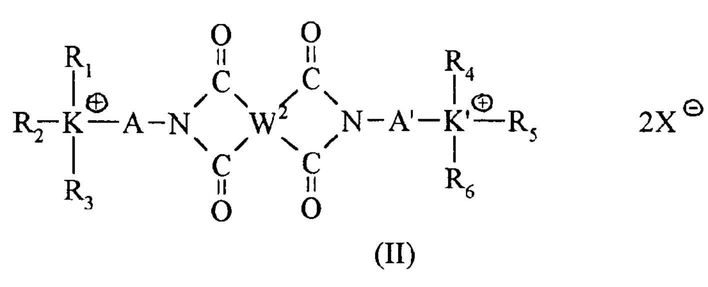

(II)

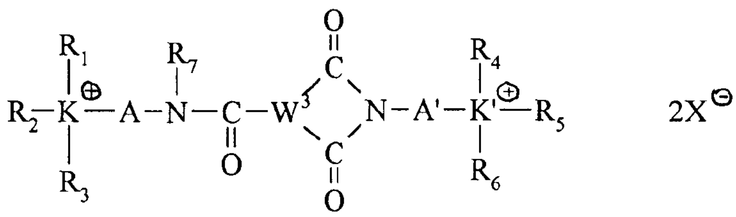

(III)

dans lesquelles

$R_1$ à $R_8$ représentent indépendamment les uns des autres un atome d'hydrogène ou un reste hydrocarboné qui peut être interrompu par des hétéroatomes, les restes $R_1$ et $R_2$ ou $R_4$ et $R_5$ en liaison respectivement avec K ou K′ peuvent indépendamment les uns des autres se combiner pour former un système cyclique et, dans le cas où $R_1$ ou $R_2$, ou $R_4$ ou $R_5$ sont à ce propos liés par une double liaison respectivement à K ou K′, $R_3$ ou $R_8$ deviennent sans objet, et/ou un des restes $R_1$, $R_2$ ou $R_3$ peut se combiner avec $R_7$ ou l'un des restes $R_4$, $R_8$ ou $R_8$ peut se combiner avec $R_8$ pour former un pont aliphatique de 2 à 5 atomes de carbone,

A et A′, ainsi que $W^1$, $W^2$ et $W^3$ représentent indépendamment les uns des autres un chaînon à base d'un hydrocarbure qui peut être interrompu par des hétéroatomes, $W^1$ étant un chaînon bivalent, $W^2$ un chaînon tétravalent et $W^3$ un chaînon trivalent, et $W^1$ dans le cas de la formule générale (I) peut aussi représenter une liaison directe,

K et K′ peuvent représenter indépendamment l'un de l'autre un atome d'azote, de phosphore, d'arsenic ou d'antimoine,

et l'anion $X^{\ominus}$ est l'équivalent stoechiométrique d'un ou plusieurs anions organiques ou inorganiques, mixtes ou non, le composé pouvant aussi se trouver sous forme d'un cristal mixte avec différents cations des formules générales (I) à (III),

individuellement ou en combinaison, comme agents de réglage de charge dans des toners et des révélateurs qui sont utilisés pour la copie ou la polycopie électrophotographique de modèles et pour l'impression d'informations mémorisées par un moyen électronique, optique ou magnétique ou pour la production d'épreuves en couleurs, ainsi que comme agents de réglage de charge pour des poudres et des peintures en poudre.

2. Utilisation de dérivés d'amides et d'imides dicationiques selon la revendication 1, caractérisée en ce que, dans les formules (I) à (III) citées dans la revendication 1, K et K′ représentent chacun un atome d'azote.

3. Utilisation de dérivés d'amides et d'imides dicationiques selon au moins l'une des revendications 1 et 2, caractérisée en ce que, dans les formules (I) à (III) citées dans la revendication 1, $R_1$ à $R_8$ représentent indépendamment les uns des autres des atomes d'hydrogène, des groupes alkyle linéaires ou ramifiés, saturés ou insaturés de 1 à 30 atomes de carbone, des groupes polyoxyalkylène de formule générale -(alkylène en $C_1$-$C_5$-O)$_n$-R dans laquelle R est un atome d'hydrogène, un groupe alkyle en $C_1$-$C_4$ ou un groupe acyle et n est un nombre de 1 à 10, un reste cycloaliphatique mono- ou polycyclique de 5 à 12 atomes de carbone, un reste aromatique mono- ou polycyclique ou un reste araliphatique, ces restes aliphatiques, cycloaliphatiques, araliphatiques et aromatiques pouvant être substitués par des groupes acide carboxylique et/ou sulfonique ou leurs sels, amides ou esters, des groupes hydroxy, alkyle en $C_1$-$C_4$, alcoxy en $C_1$-$C_4$, des groupes amino primaires, secondaires ou tertiaires, ainsi que par des atomes de fluor, de chlore ou de brome, de préférence par des atomes de fluor pour les restes aliphatiques, et lesdits systèmes cycliques aliphatiques, cycloaliphatiques, araliphatiques ou aromatiques pouvant contenir un ou plusieurs hétéroatomes et, indépendamment les uns des autres, $R_1$ et $R_2$, ou $R_4$ et $R_5$ en liaison respectivement avec K ou K′ peuvent se combiner pour former un système cyclique de 5 à 7 chaînons saturé ou insaturé, aromatique ou non aromatique, qui peut contenir d'autres hétéroatomes et être substitué et/ou modifié par condensation ou par pontage pour former d'autres systèmes cycliques, et, dans le cas où $R_1$ ou $R_2$, ou $R_4$ ou $R_8$ sont respectivement liés par une double liaison à K ou K′, $R_3$ ou $R_8$ deviennent sans objet.

4. Utilisation de dérivés d'amides et d'imides dicationiques selon au moins l'une des revendications 1 à 3, caractérisée en ce que, dans les formules (I) à (III) citées dans la revendication 1, A et A′ représentent indépendamment l'un de l'autre un chaînon aliphatique linéaire ou ramifié, saturé ou insaturé, de 1 à 30

atomes de carbone, un chaînon cycloaliphatique mono- ou polycyclique, un chaînon aromatique mono- ou polycyclique ou un chaînon araliphatique, les chaînons aliphatiques, cycloaliphatiques, araliphatiques et aromatiques pouvant être substitués par des groupes hydroxy, acide carboxylique et/ou sulfonique ou leurs sels, amides ou esters, des groupes alkyle en $C_1$-$C_4$, alcoxy en $C_1$-$C_4$, des groupes amino primaires, secondaires ou tertiaires, ainsi que par des atomes de fluor, de chlore ou de brome, de préférence par des atomes de fluor pour les chaînons aliphatiques, et lesdits systèmes cycliques aliphatiques, cycloaliphatiques, aromatiques et araliphatiques pouvant contenir un ou plusieurs atomes d'azote et/ou d'oxygène et/ou de soufre.

5. Utilisation de dérivés d'amides et d'imides dicationiques selon au moins l'une des revendications 1 à 4, caractérisée en ce que, dans les formules (I) à (III) citées dans la revendication 1,

$W^1$ représente en tant que chaînon divalent un chaînon aliphatique linéaire ou ramifié, saturé ou insaturé de 1 à 30 atomes de carbone, un chaînon polyoxyalkylène de formule générale $-CH_2-O-$(alkylène en $C_1$-$C_5$ - $O)_m-CH_2-$, dans laquelle m est un nombre de O à 10, un chaînon cycloaliphatique mono- ou polycyclique de 5 à 12 atomes de carbone, un chaînon aromatique mono- ou polycyclique ou un chaînon araliphatique, les chaînons aliphatiques, cycloaliphatiques, araliphatiques et aromatiques pouvant être substitués par des groupes acide carboxylique et/ou sulfonique ou leurs sels, amides ou esters, des groupes hydroxy, alkyle en $C_1$-$C_4$, alcoxy en $C_1$-$C_4$, des groupes amino primaires, secondaires ou tertiaires ainsi que par des atomes de fluor, de chlore ou de brome, de préférence par des atomes de fluor pour les chaînons aliphatiques, et les chaînons intermédiaires aliphatiques ainsi que les systèmes cycliques cycloaliphatiques, araliphatiques et aromatiques pouvant contenir un ou plusieurs atomes d'azote et/ou d'oxygène et/ou de soufre, et $W^1$ peut aussi représenter une liaison directe,

$W^2$ en tant que chaînon tétravalent représente un chaînon aliphatique linéaire ou ramifié, saturé ou insaturé de 2 à 30 atomes de carbone, un chaînon aromatique, cycloaliphatique, mono- ou polycyclique de 5 à 12 atomes de carbone ou un chaînon araliphatique, les chaînons aliphatiques, cycloaliphatiques, araliphatiques et aromatiques pouvant être substitués par des groupes acide carboxylique et/ou sulfonique ou leurs sels, amides ou esters, des groupes hydroxy, alkyle en $C_1$-$C_4$, alcoxy en $C_1$-$C_4$, des groupes amino primaires, secondaires ou tertiaires ainsi que par des atomes de fluor, de chlore ou de brome, de préférence par des atomes de fluor pour les chaînons aliphatiques, et les chaînons aliphatiques ainsi que les systèmes cycliques cycloaliphatiques, araliphatiques et aromatiques pouvant contenir un ou plusieurs atomes d'azote et/ou d'oxygène et/ou de soufre,

et $W^3$ en tant que chaînon trivalent représente un chaînon aliphatique linéaire ou ramifié, saturé ou insaturé de 2 à 30 atomes de carbone, un chaînon cycloaliphatique mono- ou polycyclique de 5 à 12 atomes de carbone, un chaînon aromatique mono- ou polycyclique ou un chaînon araliphatique, les chaînons aliphatiques, araliphatiques et aromatiques pouvant être substitués par des groupes acide carboxylique et/ou sulfonique ou leurs sels, amides ou esters, des groupes hydroxy, alkyle en $C_1$-$C_4$, alcoxy en $C_1$-$C_4$, des groupes amino primaires, secondaires ou tertiaires ainsi que par des atomes de fluor, de chlore ou de brome, de préférence par des atomes de fluor pour les chaînons aliphatiques, et les chaînons aliphatiques ainsi que les systèmes cycliques cycloaliphatiques, araliphatiques et aromatiques pouvant contenir un ou plusieurs atomes d'azote et/ou d'oxygène et/ou de soufre.

6. Utilisation de dérivés d'amides et d'imides dicationiques selon au moins l'une des revendications 1 à 5, caractérisée en ce que, dans les formules (I) à (III) citées dans la revendication 1, l'anion $X^{\ominus}$ représente un halogénure, $PF_6^{\ominus}$, $SO_4^{2\ominus}$, $HSO_4^{\ominus}$, phosphate, $NO_3^{\ominus}$, cyanate, thiocyanate, $BF_4^{\ominus}$, $B(aryl)_4^{\ominus}$, phénolate, nitrophénolate, tétracyanate de zinc, tétrathiocyanate de zinc, $CH_3OSO_3^{\ominus}$, $CH_2H_5OSO_3^{\ominus}$, un carboxylate ou sulfonate saturé ou insaturé aliphatique ou aromatique, ou un anion tungsténate, molybdate, ou un anion d'hétéropolyacide, ces anions pouvant aussi se trouver sous forme mixte.

7. Utilisation de dérivés d'amides et d'imides dicationiques selon au moins l'une des revendications 1 à 6, caractérisée en ce que, dans les formules (I) à (III) citées dans la revendication 1,

K et K' représentent chacun un atome d'azote,

$R_1$ à $R_6$ représentent chacun, indépendamment les uns des autres, un atome d'hydrogène ou un groupe alkyle en $C_1$-$C_4$ ou $R_1$ et $R_2$ ou $R_4$ et $R_5$ peuvent indépendamment les uns des autres se combiner pour former respectivement avec K et K' un système hétérocyclique saturé ou insaturé de 5 ou 6 chaînons contenant un ou deux atomes d'azote comme hétéroatomes, et $R_3$ et/ou $R_5$ représentent alors indépendamment l'un de l'autre un atome d'hydrogène ou un groupe alkyle en $C_1$-$C_4$, ou, dans le cas où $R_1$ ou $R_2$ , ou $R_4$ ou $R_5$ forment à ce propos une double liaison avec respectivement K ou K', $R_3$ et/ou $R_5$ sont sans objet,

$R_7$ et $R_8$ représentent un atome d'hydrogène, et

A et A′ représentent chacun, indépendamment l'un de l'autre, un chaînon $(CH_2)_p$ avec p = 1 à 4, un chaînon phénylènen, naphtylène ou

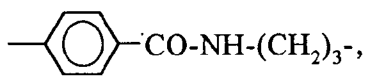

$W^1$ représente un chaînon phénylène, naphtylène, cyclohexylène, $(CH_2)_q$, avec q = 1 à 12, ou un chaînon $(CH_2\text{-}O(CH_2\text{-}CH_2\text{-}O)_r\text{-}CH_2\text{-}$ avec r = 1 à 4,

$W^2$ représente un chaînon phénylène ou naphtylène, les groupes carboxyle nécessaires pour la formation de l'imide étant en position ortho l'un par rapport à l'autre dans le cas du phénylène et en position ortho et/ou péri dans le cas du naphtylène, ou un chaînon éthylènediaminetétraméthylène,

et $W^3$ est un chaînon phénylène ou naphtylène, et

l'anion $X^{\ominus}$ représente un anion $B(aryle)_4^{\ominus}$, $BF_4^{\ominus}$, $PF_6^{\ominus}$, $SCN^{\ominus}$ ou $CH_3SO_4^{\ominus}$.

**8.** Utilisation de dérivés d'amides et d'imides dicationiques selon au moins l'une des revendications 1 à 7, caractérisée en ce que l'on utilise ces composés individuellement ou en combinaison à une concentration d'environ 0,01 à environ 30 % en masse.

**9.** Utilisation d'au moins un des composés cités dans les revendications 1 à 7 comme constituant de revêtements de supports qui sont utilisés dans des révélateurs pour la copie ou la polycopie de modèles et pour l'impression d'informations mémorisées par un moyen électronique, optique ou magnétique, ou dans la production d'épreuves en couleurs.

**10.** Utilisation des composés cités dans au moins une des revendications 1 à 7, individuellement ou en combinaison, comme agents améliorant la charge dans des poudres et des peintures pour le revêtement de surface d'objets en métal, bois, matière plastique, verre, céramique, béton, textile, papier ou caoutchouc, en particulier dans des peintures en poudre pulvérisées par un moyen triboélectrique ou électrocinétique.